# EUROPEAN PATENT SPECIFICATION

(11) **EP 0 614 979 B1**
(45) Date of publication and mention of the grant of the patent: **05.06.1996**
(21) Application number: 94200200.7
(22) Date of filing: 12.03.1990
(51) Int. Cl.: C12N 15/40, C07K 14/185, A61K 39/187, C12Q 1/70

(54) **Hog cholera virus vaccine and diagnostic**
Impfstoff und Diagnostikum für den Schweine-Cholera-Virus
Vaccin et test de diagnostique pour le virus du choléra porcin

(30) Priority: 19.03.1989 EP 89104921
(43) Date of publication of application: 14.09.1994
(62) Divisional of application: 90200573.5
(73) Proprietor: Akzo Nobel N.V., NL-6824 BM Arnhem (NL)
(72) Inventor: Meyers, Gregor, D-72141 Walddorfhaslach (DE); Rümenapf, Tillmann, D-72076 Tubingen (DE); Thiel, Heinz-Jürgen, D-72076 Tübingen (DE)
(74) Representative: Mestrom, Joannes Jozef Louis

(56) References cited:
- EP-A- 0 236 977
- VIRUS RESEARCH vol. 11, no. 4 , 1988 , AMSTERDAM, NL pages 281 - 291 MOORMANN AND HULST 'Hog cholera virus: identification and characterization of the viral RNA and the virus-specific RNA synthesized in infected swine kidney cells'
- VIROLOGY vol. 171, no. 1 , July 1989 pages 18 - 27 RÜMENAPF ET AL. 'Hog Choler Virus-Characterization of Specific Antiserum and Identification of cDNA Clones'
- VIROLOGY vol. 171, no. 1 , July 1989 pages 18 - 27 RÜMENAPF ET AL. 'Hog Cholera Virus Characterization of Specific Antiserum and Identification of cDNA Clones'

### SEQUENCE LISTING

(1) GENERAL INFORMATION:
   (i) APPLICANT:
      (A) NAME: Akzo N.V.
      (B) STREET: Velperweg 76
      (C) CITY: Arnhem
      (E) COUNTRY: The Netherlands
      (F) POSTAL CODE (ZIP): 6824 BM
      (G) TELEPHONE: 04120-66379
      (H) TELEFAX: 04120-50592
      (I) TELEX: 37503 akpha nl
   (ii) TITLE OF INVENTION: Hog cholera virus vaccine and diagnostic
   (iii) NUMBER OF SEQUENCES: 9
   (v) COMPUTER READABLE FORM:
      (A) MEDIUM TYPE: Floppy disk
      (B) COMPUTER: IBM PC compatible
      (C) OPERATING SYSTEM: PC-DOS/MS-DOS
      (D) SOFTWARE: PatentIn Release #1.0, Version #1.25
(2) INFORMATION FOR SEQ ID NO:1:
   (i) SEQUENCE CHARACTERISTICS:
      (A) LENGTH: 12284 base pairs
      (B) TYPE: nucleic acid
      (C) STRANDEDNESS: double
      (D) TOPOLOGY: linear
   (ii) MOLECULE TYPE: cDNA
   (vi) ORIGINAL SOURCE:
      (A) ORGANISM: Hog cholera virus
      (B) STRAIN: Alfort
      (H) CELL LINE: PK 15 and 38A1D
   (ix) FEATURE:
      (A) NAME/KEY: CDS
      (B) LOCATION: 364..12060
      (D) OTHER INFORMATION: /label= 435_kDA_protein
   (ix) FEATURE:
      (A) NAME/KEY: primer_bind
      (B) LOCATION: complement (2587..2619)
      (D) OTHER INFORMATION: /label= primer_1
   (ix) FEATURE:
      (A) NAME/KEY: primer_bind
      (B) LOCATION: complement (2842..2880)
      (D) OTHER INFORMATION: /label= primer_2
   (ix) FEATURE:
      (A) NAME/KEY: variation
      (B) LOCATION: replace (127, "c")
   (ix) FEATURE:
      (A) NAME/KEY: variation
      (B) LOCATION: replace (1522, "g")
   (ix) FEATURE:
      (A) NAME/KEY: variation
      (B) LOCATION: replace(10989, "t")
   (xi) SEQUENCE DESCRIPTION: SEQ ID NO:1:
   GTTAGCTCTT TCTCGTATAC GATATTGGAT ACACTAAATT TCGATTTGGT CTAGGGCACC  60
   CCTCCAGCGA CGGCCGAAAT GGGCTAGCCA TGCCCATAGT AGGACTAGCA AACGGAGGGA 120
   CTAGCCGTAG TGGCGAGCTC CCTGGGTGGT CTAAGTCCTG AGTACAGGAC AGTCGTCAGT 180
   AGTTCGACGT GAGCACTAGC CCACCTCGAG ATGCTACGTG GACGAGGGCA TGCCCAAGAC 240
   ACACCTTAAC CCTGGCGGGG GTCGCTAGGG TGAAATCACA TTATGTGATG GGGGTACGAC 300
   CTGATAGGGT GCTGCAGAGG CCCACTAGCA GGCTAGTATA AAAATCTCTG CTGTACATGG 360
   CAC ATG GAG TTG AAT CAT TTT GAA TTA TTA TAC AAA ACA AGC AAA CAA    408
       Met Glu Leu Asn His Phe Glu Leu Leu Tyr Lys Thr Ser Lys Gln
         1               5                  10                  15
   AAA CCA GTG GGA GTG GAG GAA CCG GTG TAT GAC ACC GCG GGG AGA CCA    456
   Lys Pro Val Gly Val Glu Glu Pro Val Tyr Asp Thr Ala Gly Arg Pro
                    20                  25                  30
   CTA TTT GGG AAC CCA AGT GAG GTA CAC CCA CAA TCA ACG CTG AAG CTG    504
   Leu Phe Gly Asn Pro Ser Glu Val His Pro Gln Ser Thr Leu Lys Leu
                35                  40                  45
   CCA CAC GAC AGG GGG AGA GGA GAT ATC AGA ACA ACA CTG AGG GAC CTA    552
   Pro His Asp Arg Gly Arg Gly Asp Ile Arg Thr Thr Leu Arg Asp Leu
            50                  55                  60
   CCC AGG AAA GGT GAC TGT AGG AGT GGC AAC CAT CTA GGC CCG GTT AGT    600
   Pro Arg Lys Gly Asp Cys Arg Ser Gly Asn His Leu Gly Pro Val Ser
        65                  70                  75
   GGG ATA TAC ATA AAG CCC GGC CCT GTC TAC TAT CAG GAC TAC ACG GGC    648
   Gly Ile Tyr Ile Lys Pro Gly Pro Val Tyr Tyr Gln Asp Tyr Thr Gly
    80                  85                  90                  95
   CCA GTC TAT CAC AGA GCT CCT TTA GAG TTC TTT GAT GAG GCC CAG TTC    696
   Pro Val Tyr His Arg Ala Pro Leu Glu Phe Phe Asp Glu Ala Gln Phe
                   100                 105                 110
   TGC GAG GTG ACT AAG AGA ATA GGC AGG GTC ACG GGT AGT GAT GGT AAG     744
   Cys Glu Val Thr Lys Arg Ile Gly Arg Val Thr Gly Ser Asp Gly Lys
               115                 120                 125
   CTT TAC CAC ATA TAT GTG TGC GTC GAT GGT TGC ATA CTG CTG AAA TTA     792
   Leu Tyr His Ile Tyr Val Cys Val Asp Gly Cys Ile Leu Leu Lys Leu
           130                 135                 140
   GCC AAA AGG GGC ACA CCC AGA ACC CTA AAG TGG ATT AGG AAC TTC ACC     840
   Ala Lys Arg Gly Thr Pro Arg Thr Leu Lys Trp Ile Arg Asn Phe Thr
       145                 150                 155
   AAC TGT CCA TTA TGG GTA ACC AGT TGC TCC GAT GAC GGC GCA AGT GGC     888
   Asn Cys Pro Leu Trp Val Thr Ser Cys Ser Asp Asp Gly Ala Ser Gly
   160                 165                 170                 175
   AGC AAG GAT AAG AAG CCA GAC AGA ATG AAC AAA GGT AAG TTG AAG ATA     936
   Ser Lys Asp Lys Lys Pro Asp Arg Met Asn Lys Gly Lys Leu Lys Ile
                   180                 185                 190
   GCC CCA AGA GAG CAT GAG AAG GAC AGC AAG ACC AAG CCT CCT GAT GCA     984
   Ala Pro Arg Glu His Glu Lys Asp Ser Lys Thr Lys Pro Pro Asp Ala
               195                 200                 205
   ACG ATT GTA GTA GAG GGA GTA AAA TAC CAA ATC AAA AAG AAA GGC AAA    1032
   Thr Ile Val Val Glu Gly Val Lys Tyr Gln Ile Lys Lys Lys Gly Lys
           210                 215                 220
   GTC AAA GGG AAG AAC ACA CAA GAC GGC CTG TAC CAT AAT AAG AAC AAG    1080
   Val Lys Gly Lys Asn Thr Gln Asp Gly Leu Tyr His Asn Lys Asn Lys
       225                 230                 235
   CCA CCA GAG TCC AGG AAG AAA CTA GAA AAA GCC CTG TTG GCT TGG GCG    1128
   Pro Pro Glu Ser Arg Lys Lys Leu Glu Lys Ala Leu Leu Ala Trp Ala
   240                 245                 250                 255
   GTG ATA ACA ATC TTG CTG TAC CAG CCT GTA GCA GCC GAG AAC ATA ACT    1176
   Val Ile Thr Ile Leu Leu Tyr Gln Pro Val Ala Ala Glu Asn Ile Thr
                   260                 265                 270
   CAA TGG AAC CTG AGT GAC AAC GGC ACT AAT GGT ATT CAG CGA GCC ATG    1224
   Gln Trp Asn Leu Ser Asp Asn Gly Thr Asn Gly Ile Gln Arg Ala Met
               275                 280                 285
   TAT CTT AGA GGG GTT AAC AGG AGC TTA CAT GGG ATC TGG CCC GAG AAA    1272
   Tyr Leu Arg Gly Val Asn Arg Ser Leu His Gly Ile Trp Pro Glu Lys
           290                 295                 300
   ATA TGC AAG GGG GTC CCC ACT CAT CTG GCC ACT GAC ACG GAA CTG AAA    1320
   Ile Cys Lys Gly Val Pro Thr His Leu Ala Thr Asp Thr Glu Leu Lys
       305                 310                 315
   GAG ATA CGC GGG ATG ATG GAT GCC AGC GAG AGG ACA AAC TAT ACG TGC    1368
   Glu Ile Arg Gly Met Met Asp Ala Ser Glu Arg Thr Asn Tyr Thr Cys
   320                 325                 330                 335
   TGT AGG TTA CAA AGA CAT GAA TGG AAC AAA CAT GGA TGG TGT AAC TGG   1416
   Cys Arg Leu Gln Arg His Glu Trp Asn Lys His Gly Trp Cys Asn Trp
                   340                 345                 350
   TAC AAC ATA GAC CCT TGG ATT CAG TTA ATG AAC AGG ACC CAA ACA AAT   1464
   Tyr Asn Ile Asp Pro Trp Ile Gln Leu Met Asn Arg Thr Gln Thr Asn
               355                 360                 365
   TTG ACA GAA GGC CCT CCA GAT AAG GAG TGT GCC GTG ACC TGC AGG TAT   1512
   Leu Thr Glu Gly Pro Pro Asp Lys Glu Cys Ala Val Thr Cys Arg Tyr
           370                 375                 380
   GAC AAA AAT ACC GAT GTC AAC GTG GTC ACC CAG GCC AGG AAT AGG CCA   1560
   Asp Lys Asn Thr Asp Val Asn Val Val Thr Gln Ala Arg Asn Arg Pro
       385                 390                 395
   ACT ACT CTG ACT GGC TGC AAG AAA GGG AAA AAC TTT TCA TTC GCA GGC   1608
   Thr Thr Leu Thr Gly Cys Lys Lys Gly Lys Asn Phe Ser Phe Ala Gly
   400                 405                 410                 415
   ACA GTC ATA GAG GGC CCG TGC AAT TTC AAC GTT TCC GTG GAG GAC ATC   1656
   Thr Val Ile Glu Gly Pro Cys Asn Phe Asn Val Ser Val Glu Asp Ile
                   420                 425                 430
   TTA TAC GGA GAC CAT GAG TGT GGC AGT CTG CTC CAG GAC ACG GCT CTG   1704
   Leu Tyr Gly Asp His Glu Cys Gly Ser Leu Leu Gln Asp Thr Ala Leu
               435                 440                 445
   TAC CTA TTG GAT GGA ATG ACC AAC ACT ATA GAG AAT GCC AGG CAA GGT   1752
   Tyr Leu Leu Asp Gly Met Thr Asn Thr Ile Glu Asn Ala Arg Gln Gly
           450                 455                 460
   GCG GCG CGG GTG ACA TCT TGG CTT GGG AGG CAG CTC AGT ACC GCA GGG   1800
   Ala Ala Arg Val Thr Ser Trp Leu Gly Arg Gln Leu Ser Thr Ala Gly
       465                 470                 475
   AAG AAG CTA GAG AGG AGA AGC AAA ACC TGG TTT GGT GCC TAT GCC CTG   1848
   Lys Lys Leu Glu Arg Arg Ser Lys Thr Trp Phe Gly Ala Tyr Ala Leu
   480                 485                 490                 495
   TCA CCT TAC TGC AAT GTG ACT AGA AAA ATA GGG TAC ATA TGG TAT ACA   1896
   Ser Pro Tyr Cys Asn Val Thr Arg Lys Ile Gly Tyr Ile Trp Tyr Thr
                   500                 505                 510
   AAC AAC TGC ACC CCG GCA TGC CTC CCT AAG AAC ACA AAA ATA ATA GGC   1944
   Asn Asn Cys Thr Pro Ala Cys Leu Pro Lys Asn Thr Lys Ile Ile Gly
               515                 520                 525
   CCT GGA AAG TTT GAC ACC AAT GCG GAA GAC GGG AAG ATC CTT CAT GAA   1992
   Pro Gly Lys Phe Asp Thr Asn Ala Glu Asp Gly Lys Ile Leu His Glu
           530                 535                 540
   ATG GGG GGC CAC CTA TCA GAA TTT TTG TTG CTT TCT CTA GTT ATC CTG   2040
   Met Gly Gly His Leu Ser Glu Phe Leu Leu Leu Ser Leu Val Ile Leu
       545                 550                 555
   TCT GAC TTT GCC CCC GAG ACA GCT AGC ACG CTA TAC CTA ATT TTA CAC   2088
   Ser Asp Phe Ala Pro Glu Thr Ala Ser Thr Leu Tyr Leu Ile Leu His
   560                 565                 570                 575
   TAT GCA ATC CCC CAG TCC CAC GAA GAA CCT GAA GGT TGT GAT ACG AAC   2136
   Tyr Ala Ile Pro Gln Ser His Glu Glu Pro Glu Gly Cys Asp Thr Asn
                   580                 585                 590
   CAA CTT AAC CTA ACA GTG AAA CTT AGG ACA GAA GAC GTA GTG CCA TCA   2184
   Gln Leu Asn Leu Thr Val Lys Leu Arg Thr Glu Asp Val Val Pro Ser
               595                 600                 605
   TCA GTT TGG AAT ATT GGC AAA TAT GTT TGT GTT AGA CCA GAC TGG TGG   2232
   Ser Val Trp Asn Ile Gly Lys Tyr Val Cys Val Arg Pro Asp Trp Trp
           610                 615                 620
   CCG TAT GAA ACT AAA GTG GCT CTG CTG TTT GAA GAG GCA GGA CAG GTT   2280
   Pro Tyr Glu Thr Lys Val Ala Leu Leu Phe Glu Glu Ala Gly Gln Val
       625                 630                 635
   ATA AAG CTA GTC CTA CGG GCA CTG AGG GAT TTA ACT AGG GTC TGG AAC   2328
   Ile Lys Leu Val Leu Arg Ala Leu Arg Asp Leu Thr Arg Val Trp Asn
   640                 645                 650                 655
   AGC GCA TCA ACT ACT GCG TTT CTC ATT TGC TTG ATA AAA GTA TTG AGA   2376
   Ser Ala Ser Thr Thr Ala Phe Leu Ile Cys Leu Ile Lys Val Leu Arg
                   660                 665                 670
   GGA CAG GTT GTG CAA GGT ATA ATA TGG CTG CTG CTG GTG ACC GGG GCA   2424
   Gly Gln Val Val Gln Gly Ile Ile Trp Leu Leu Leu Val Thr Gly Ala
               675                 680                 685
   CAA GGG CGG CTA GCC TGT AAG GAA GAC TAC AGG TAT GCG ATC TCG TCA   2472
   Gln Gly Arg Leu Ala Cys Lys Glu Asp Tyr Arg Tyr Ala Ile Ser Ser
           690                 695                 700
   ACC AAT GAG ATA GGG CTG CTG GGC GCT GAA GGT CTC ACC ACT ACC TGG   2520
   Thr Asn Glu Ile Gly Leu Leu Gly Ala Glu Gly Leu Thr Thr Thr Trp
       705                 710                 715
   AAA GAA TAC AGC CAC GGT TTG CAG CTG GAC GAC GGA ACC GTT AAG GCC   2568
   Lys Glu Tyr Ser His Gly Leu Gln Leu Asp Asp Gly Thr Val Lys Ala
   720                 725                 730                 735
   GTC TGC ACT GCA GGG TCC TTT AAA GTC ACA GCA CTT AAC GTG GTT AGT   2616
   Val Cys Thr Ala Gly Ser Phe Lys Val Thr Ala Leu Asn Val Val Ser
                   740                 745                 750
   AGG AGG TAT CTA GCA TCA TTG CAC AAG AGG GCT CTA CCC ACC TCA GTG   2664
   Arg Arg Tyr Leu Ala Ser Leu His Lys Arg Ala Leu Pro Thr Ser Val
               755                 760                 765
   ACA TTT GAG CTC CTA TTT GAC GGG ACC AAC CCA GCA ATC GAG GAG ATG   2712
   Thr Phe Glu Leu Leu Phe Asp Gly Thr Asn Pro Ala Ile Glu Glu Met
           770                 775                 780
   GAT GAT GAC TTC GGA TTT GGG CTG TGC CCA TTT GAC ACG AGT CCT GTG   2760
   Asp Asp Asp Phe Gly Phe Gly Leu Cys Pro Phe Asp Thr Ser Pro Val
       785                 790                 795
   ATC AAA GGG AAG TAC AAC ACC ACT TTG TTA AAC GGC AGT GCT TTC TAT   2808
   Ile Lys Gly Lys Tyr Asn Thr Thr Leu Leu Asn Gly Ser Ala Phe Tyr
   800                 805                 810                 815
   CTA GTC TGC CCA ATA GGA TGG ACT GGT GTC GTA GAG TGC ACA GCA GTG   2856
   Leu Val Cys Pro Ile Gly Trp Thr Gly Val Val Glu Cys Thr Ala Val
                   820                 825                 830
   AGC CCC ACA ACC TTG AGA ACA GAA GTG GTG AAA ACC TTC AGG AGA GAT   2904
   Ser Pro Thr Thr Leu Arg Thr Glu Val Val Lys Thr Phe Arg Arg Asp
               835                 840                 845
   AAG CCT TTT CCA CAT AGA GTA GAC TGT GTG ACC ACC ATA GTA GAA AAA   2952
   Lys Pro Phe Pro His Arg Val Asp Cys Val Thr Thr Ile Val Glu Lys
           850                 855                 860
   GAA GAC CTA TTC CAT TGC AAG TTG GGG GGT AAT TGG ACA TGT GTA AAA   3000
   Glu Asp Leu Phe His Cys Lys Leu Gly Gly Asn Trp Thr Cys Val Lys
       865                 870                 875
   GGC GAC CCA GTG ACT TAT AAG GGG GGG CAA GTA AAG CAG TGC AGG TGG   3048
   Gly Asp Pro Val Thr Tyr Lys Gly Gly Gln Val Lys Gln Cys Arg Trp
   880                 885                 890                 895
   TGT GGT TTC GAG TTT AAA GAG CCC TAC GGG CTC CCA CAC TAC CCT ATA   3096
   Cys Gly Phe Glu Phe Lys Glu Pro Tyr Gly Leu Pro His Tyr Pro Ile
                   900                 905                 910
   GGC AAG TGC ATC CTA ACA AAT GAG ACA GGT TAC AGG GTA GTA GAT TCC   3144
   Gly Lys Cys Ile Leu Thr Asn Glu Thr Gly Tyr Arg Val Val Asp Ser
               915                 920                 925
   ACA GAC TGC AAC AGA GAT GGC GTC GTT ATT AGC ACT GAA GGG GAA CAT   3192
   Thr Asp Cys Asn Arg Asp Gly Val Val Ile Ser Thr Glu Gly Glu His
           930                 935                 940
   GAG TGC TTG ATT GGC AAC ACT ACC GTC AAG GTG CAT GCA CTG GAT GAA   3240
   Glu Cys Leu Ile Gly Asn Thr Thr Val Lys Val His Ala Leu Asp Glu
       945                 950                 955
   AGA TTG GGC CCT ATG CCG TGC AGA CCC AAA GAA ATC GTC TCT AGT GAG   3288
   Arg Leu Gly Pro Met Pro Cys Arg Pro Lys Glu Ile Val Ser Ser Glu
   960                 965                 970                 975
   GGA CCT GTG AGG AAA ACT TCT TGT ACA TTC AAC TAC ACA AAG ACT CTA   3336
   Gly Pro Val Arg Lys Thr Ser Cys Thr Phe Asn Tyr Thr Lys Thr Leu
                   980                 985                 990
   AGA AAC AAA TAC TAT GAG CCC AGA GAC AGT TAC TTC CAG CAA TAT ATG   3384
   Arg Asn Lys Tyr Tyr Glu Pro Arg Asp Ser Tyr Phe Gln Gln Tyr Met
               995                1000                1005
   CTC AAG GGC GAG TAT CAA TAC TGG TTT AAT CTG GAC GTG ACC GAC CAC   3432
   Leu Lys Gly Glu Tyr Gln Tyr Trp Phe Asn Leu Asp Val Thr Asp His
           1010                1015                1020
   CAC ACA GAC TAC TTT GCC GAG TTT GTT GTC TTG GTA GTA GTA GCA CTG   3480
   His Thr Asp Tyr Phe Ala Glu Phe Val Val Leu Val Val Val Ala Leu
       1025                1030                1035
   TTA GGA GGA AGG TAC GTT CTG TGG CTA ATA GTG ACC TAC ATA ATT CTA   3528
   Leu Gly Gly Arg Tyr Val Leu Trp Leu Ile Val Thr Tyr Ile Ile Leu
   1040                1045                1050                1055
   ACA GAG CAG CTC GCT GCT GGT CTA CAG CTA GGC CAG GGT GAG GTG GTA   3576
   Thr Glu Gln Leu Ala Ala Gly Leu Gln Leu Gly Gln Gly Glu Val Val
                   1060                1065                1070
   TTG ATA GGG AAC CTA ATT ACC CAC ACG GAC AAT GAG GTG GTG GTG TAC   3624
   Leu Ile Gly Asn Leu Ile Thr His Thr Asp Asn Glu Val Val Val Tyr
               1075                1080                1085
   TTC CTA CTG CTC TAC TTA GTA ATA AGA GAT GAG CCC ATA AAG AAA TGG   3672
   Phe Leu Leu Leu Tyr Leu Val Ile Arg Asp Glu Pro Ile Lys Lys Trp
           1090                1095                1100
   ATA CTA CTG CTG TTT CAT GCA ATG ACT AAC AAT CCA GTC AAG ACC ATA   3720
   Ile Leu Leu Leu Phe His Ala Met Thr Asn Asn Pro Val Lys Thr Ile
       1105                1110                1115
   ACA GTA GCA TTG CTA ATG ATC AGT GGG GTT GCC AAG GGT GGT AAG ATA   3768
   Thr Val Ala Leu Leu Met Ile Ser Gly Val Ala Lys Gly Gly Lys Ile
   1120                1125                1130                1135
   GAT GGT GGC TGG CAG AGA CAA CCG GTG ACC AGT TTT GAC ATC CAA CTC   3816
   Asp Gly Gly Trp Gln Arg Gln Pro Val Thr Ser Phe Asp Ile Gln Leu
                   1140                1145                1150
   GCA CTG GCA GTC GTA GTA GTC GTT GTG ATG TTG CTG GCA AAG AGA GAC   3864
   Ala Leu Ala Val Val Val Val Val Val Met Leu Leu Ala Lys Arg Asp
               1155                1160                1165
   CCG ACT ACT TTC CCT TTG GTA ATC ACA GTG GCA ACC CTG AGA ACG GCC   3912
   Pro Thr Thr Phe Pro Leu Val Ile Thr Val Ala Thr Leu Arg Thr Ala
           1170                1175                1180
   AAG ATA ACC AAC GGT TTT AGC ACA GAT CTA GTC ATA GCC ACA GTG TCG   3960
   Lys Ile Thr Asn Gly Phe Ser Thr Asp Leu Val Ile Ala Thr Val Ser
       1185                1190                1195
   GCA GCT TTG TTA ACT TGG ACC TAT ATC AGC GAC TAC TAC AAA TAC AAG   4008
   Ala Ala Leu Leu Thr Trp Thr Tyr Ile Ser Asp Tyr Tyr Lys Tyr Lys
   1200                1205                1210                1215
   ACT TGG CTA CAG TAC CTC GTC AGC ACG GTG ACT GGA ATC TTC CTG ATA   4056
   Thr Trp Leu Gln Tyr Leu Val Ser Thr Val Thr Gly Ile Phe Leu Ile
                   1220                1225                1230
   AGG GTG CTG AAG GGA ATA GGC GAA TTG GAT CTG CAC GCC CCA ACC TTG  4104
   Arg Val Leu Lys Gly Ile Gly Glu Leu Asp Leu His Ala Pro Thr Leu
               1235                1240                1245
   CCG TCT CAC AGA CCC CTC TTT TAC ATC CTT GTA TAC CTT ATT TCC ACT  4152
   Pro Ser His Arg Pro Leu Phe Tyr Ile Leu Val Tyr Leu Ile Ser Thr
           1250                1255                1260
   GCC GTG GTA ACT AGA TGG AAT CTG GAC GTA GCC GGA TTG TTG CTG CAG  4200
   Ala Val Val Thr Arg Trp Asn Leu Asp Val Ala Gly Leu Leu Leu Gln
       1265                1270                1275
   TGC GTC CCA ACT CTT TTA ATG GTT TTT ACG ATG TGG GCA GAC ATT CTC  4248
   Cys Val Pro Thr Leu Leu Met Val Phe Thr Met Trp Ala Asp Ile Leu
   1280                1285                1290                1295
   ACC CTA ATT CTC ATA CTA CCT ACT TAT GAG TTA ACA AAG TTA TAC TAC  4296
   Thr Leu Ile Leu Ile Leu Pro Thr Tyr Glu Leu Thr Lys Leu Tyr Tyr
                   1300                1305                1310
   CTT AAG GAA GTG AAG ATT GGG GCA GAA AGA GGT TGG CTG TGG AAA ACT  4344
   Leu Lys Glu Val Lys Ile Gly Ala Glu Arg Gly Trp Leu Trp Lys Thr
               1315                1320                1325
   AAC TAT AAG AGG GTA AAC GAC ATC TAC GAG GTC GAC CAA ACT AGC GAA  4392
   Asn Tyr Lys Arg Val Asn Asp Ile Tyr Glu Val Asp Gln Thr Ser Glu
           1330                1335                1340
   GGG GTT TAC CTT TTC CCT TCT AAA CAG AGG ACG AGC GCT ATA ACT AGT  4440
   Gly Val Tyr Leu Phe Pro Ser Lys Gln Arg Thr Ser Ala Ile Thr Ser
       1345                1350                1355
   ACC ATG TTG CCA TTA ATC AAA GCC ATA CTC ATT AGC TGC ATC AGC AAC  4488
   Thr Met Leu Pro Leu Ile Lys Ala Ile Leu Ile Ser Cys Ile Ser Asn
   1360                1365                1370                1375
   AAG TGG CAA CTC ATA TAC TTA CTG TAC TTG ATA TTT GAA GTG TCT TAC  4536
   Lys Trp Gln Leu Ile Tyr Leu Leu Tyr Leu Ile Phe Glu Val Ser Tyr
                   1380                1385                1390
   TAC CTC CAC AAG AAA GTT ATA GAT GAA ATA GCT GGT GGG ACC AAC TTC  4584
   Tyr Leu His Lys Lys Val Ile Asp Glu Ile Ala Gly Gly Thr Asn Phe
               1395                1400                1405
   GTT TCA AGG CTC GTG GCG GCT TTG ATT GAA GTC AAT TGG GCC TTC GAC  4632
   Val Ser Arg Leu Val Ala Ala Leu Ile Glu Val Asn Trp Ala Phe Asp
           1410                1415                1420
   AAT GAA GAA GTC AAA GGC TTA AAG AAG TTC TTC TTG CTG TCT AGT AGG  4680
   Asn Glu Glu Val Lys Gly Leu Lys Lys Phe Phe Leu Leu Ser Ser Arg
       1425                1430                1435
   GTC AAA GAG TTG ATC ATC AAA CAC AAA GTG AGG AAT GAA GTA GTG GTC  4728
   Val Lys Glu Leu Ile Ile Lys His Lys Val Arg Asn Glu Val Val Val
   1440                1445                1450                1455
   CGC TGG TTT GGA GAT GAA GAG ATT TAT GGG ATG CCA AAG CTG ATC GGC   4776
   Arg Trp Phe Gly Asp Glu Glu Ile Tyr Gly Met Pro Lys Leu Ile Gly
                   1460                1465                1470
   TTA GTT AAG GCA GCA ACA CTA AGT AGA AAC AAA CAC TGT ATG TTG TGT   4824
   Leu Val Lys Ala Ala Thr Leu Ser Arg Asn Lys His Cys Met Leu Cys
               1475                1480                1485
   ACC GTC TGT GAG GAC AGA GAT TGG AGA GGG GAA ACT TGC CCT AAA TGT   4872
   Thr Val Cys Glu Asp Arg Asp Trp Arg Gly Glu Thr Cys Pro Lys Cys
           1490                1495                1500
   GGG CGT TTT GGA CCA CCA GTG GTC TGC GGT ATG ACC CTA GCC GAT TTC   4920
   Gly Arg Phe Gly Pro Pro Val Val Cys Gly Met Thr Leu Ala Asp Phe
       1505                1510                1515
   GAA GAA AAA CAC TAT AAA AGG ATT TTC ATT AGA GAG GAC CAA TCA GGC   4968
   Glu Glu Lys His Tyr Lys Arg Ile Phe Ile Arg Glu Asp Gln Ser Gly
   1520                1525                1530                1535
   GGG CCA CTT AGG GAG GAG CAT GCA GGG TAC TTG CAG TAC AAA GCC AGG   5016
   Gly Pro Leu Arg Glu Glu His Ala Gly Tyr Leu Gln Tyr Lys Ala Arg
                   1540                1545                1550
   GGT CAA CTG TTT TTG AGG AAC CTC CCA GTG TTA GCT ACA AAA GTC AAG   5064
   Gly Gln Leu Phe Leu Arg Asn Leu Pro Val Leu Ala Thr Lys Val Lys
               1555                1560                1565
   ATG CTC CTG GTT GGT AAC CTC GGG ACA GAG ATT GGG GAT CTG GAA CAC   5112
   Met Leu Leu Val Gly Asn Leu Gly Thr Glu Ile Gly Asp Leu Glu His
           1570                1575                1580
   CTT GGC TGG GTG CTT AGA GGG CCA GCT GTT TGC AAG AAG GTT ACT GAA   5160
   Leu Gly Trp Val Leu Arg Gly Pro Ala Val Cys Lys Lys Val Thr Glu
       1585                1590                1595
   CAC GAA AGA TGC ACC ACG TCT ATA ATG GAT AAG TTG ACT GCT TTC TTT   5208
   His Glu Arg Cys Thr Thr Ser Ile Met Asp Lys Leu Thr Ala Phe Phe
   1600                1605                1610                1615
   GGA GTA ATG CCA AGG GGC ACT ACT CCC AGA GCT CCC GTA AGA TTC CCT   5256
   Gly Val Met Pro Arg Gly Thr Thr Pro Arg Ala Pro Val Arg Phe Pro
                   1620                1625                1630
   ACC TCC CTC CTA AAG ATA AGA AGA GGG CTG GAG ACT GGT TGG GCT TAC   5304
   Thr Ser Leu Leu Lys Ile Arg Arg Gly Leu Glu Thr Gly Trp Ala Tyr
               1635                1640                1645
   ACA CAC CAA GGT GGC ATC AGC TCA GTA GAC CAT GTC ACT TGT GGG AAA   5352
   Thr His Gln Gly Gly Ile Ser Ser Val Asp His Val Thr Cys Gly Lys
           1650                1655                1660
   GAC TTA CTG GTG TGT GAC ACC ATG GGT CGG ACA AGG GTT GTT TGC CAG   5400
   Asp Leu Leu Val Cys Asp Thr Met Gly Arg Thr Arg Val Val Cys Gln
       1665                1670                1675
   TCA AAT AAT AAG ATG ACC GAC GAG TCC GAA TAC GGA GTC AAA ACT GAC   5448
   Ser Asn Asn Lys Met Thr Asp Glu Ser Glu Tyr Gly Val Lys Thr Asp
   1680                1685                1690                1695
   TCC GGG TGC CCA GAG GGA GCC AGG TGT TAC GTG TTT AAC CCG GAA GCA   5496
   Ser Gly Cys Pro Glu Gly Ala Arg Cys Tyr Val Phe Asn Pro Glu Ala
                   1700                1705                1710
   GTT AAC ATA TCA GGC ACT AAA GGA GCC ATG GTC CAC TTA CAG AAA ACG   5544
   Val Asn Ile Ser Gly Thr Lys Gly Ala Met Val His Leu Gln Lys Thr
               1715                1720                1725
   GGT GGA GAA TTC ACC TGT GTG ACA GCA TCA GGA ACC CCG GCC TTC TTT   5592
   Gly Gly Glu Phe Thr Cys Val Thr Ala Ser Gly Thr Pro Ala Phe Phe
           1730                1735                1740
   GAC CTC AAG AAC CTT AAG GGC TGG TCA GGG CTA CCG ATA TTT GAA GCA   5640
   Asp Leu Lys Asn Leu Lys Gly Trp Ser Gly Leu Pro Ile Phe Glu Ala
       1745                1750                1755
   TCA AGT GGA AGG GTA GTC GGA AGG GTC AAG GTC GGG AAG AAC GAG GAT   5688
   Ser Ser Gly Arg Val Val Gly Arg Val Lys Val Gly Lys Asn Glu Asp
   1760                1765                1770                1775
   TCC AAA CCA ACC AAG CTC ATG AGT GGG ATA CAA ACG GTT TCT AAA AGC   5736
   Ser Lys Pro Thr Lys Leu Met Ser Gly Ile Gln Thr Val Ser Lys Ser
                   1780                1785                1790
   GCC ACA GAC TTG ACG GAG ATG GTG AAG AAG ATA ACG ACC ATG AAC AGG   5784
   Ala Thr Asp Leu Thr Glu Met Val Lys Lys Ile Thr Thr Met Asn Arg
               1795                1800                1805
   GGA GAG TTC AGA CAA ATA ACC CTG GCC ACA GGT GCC GGA AAA ACT ACA   5832
   Gly Glu Phe Arg Gln Ile Thr Leu Ala Thr Gly Ala Gly Lys Thr Thr
           1810                1815                1820
   GAG CTC CCT AGA TCA GTT ATA GAA GAG ATA GGG AGG CAT AAG AGG GTG   5880
   Glu Leu Pro Arg Ser Val Ile Glu Glu Ile Gly Arg His Lys Arg Val
       1825                1830                1835
   TTG GTC TTA ATC CCC TTG AGG GCG GCA GCA GAA TCA GTA TAC CAA TAC   5928
   Leu Val Leu Ile Pro Leu Arg Ala Ala Ala Glu Ser Val Tyr Gln Tyr
   1840                1845                1850                1855
   ATG AGA CAG AAA CAT CCG AGT ATA GCA TTC AAT CTA AGG ATA GGT GAG   5976
   Met Arg Gln Lys His Pro Ser Ile Ala Phe Asn Leu Arg Ile Gly Glu
                   1860                1865                1870
   ATG AAG GAA GGT GAT ATG GCC ACG GGA ATA ACC TAT GCC TCT TAC GGT   6024
   Met Lys Glu Gly Asp Met Ala Thr Gly Ile Thr Tyr Ala Ser Tyr Gly
               1875                1880                1885
   TAC TTT TGC CAG ATG TCA CAA CCC AAG CTG AGA GCC GCA ATG GTA GAA   6072
   Tyr Phe Cys Gln Met Ser Gln Pro Lys Leu Arg Ala Ala Met Val Glu
           1890                1895                1900
   TAT TCC TTT ATA TTC CTA GAT GAG TAT CAT TGT GCT ACC CCA GAA CAA   6120
   Tyr Ser Phe Ile Phe Leu Asp Glu Tyr His Cys Ala Thr Pro Glu Gln
       1905                1910                1915
   CTG GCA ATC ATG GGG AAG ATC CAC AGA TTC TCA GAA AAC CTG CGG GTG   6168
   Leu Ala Ile Met Gly Lys Ile His Arg Phe Ser Glu Asn Leu Arg Val
   1920                1925                1930                1935
   GTA GCT ATG ACA GCG ACA CCG GCA GGC ACA GTA ACA ACC ACT GGG CAG   6216
   Val Ala Met Thr Ala Thr Pro Ala Gly Thr Val Thr Thr Thr Gly Gln
                   1940                1945                1950
   AAA CAC CCT ATA GAG GAA TTT ATA GCC CCG GAA GTG ATG AAA GGA GAA   6264
   Lys His Pro Ile Glu Glu Phe Ile Ala Pro Glu Val Met Lys Gly Glu
               1955                1960                1965
   GAC TTG GGT TCT GAG TAC TTA GAT ATT GCC GGA CTG AAG ATA CCA GTA   6312
   Asp Leu Gly Ser Glu Tyr Leu Asp Ile Ala Gly Leu Lys Ile Pro Val
           1970                1975                1980
   GAG GAG ATG AAG AAT AAC ATG CTA GTT TTT GTG CCC ACC AGG AAC ATG   6360
   Glu Glu Met Lys Asn Asn Met Leu Val Phe Val Pro Thr Arg Asn Met
       1985                1990                1995
   GCG GTA GAG GCG GCA AAG AAA TTG AAG GCC AAA GGA TAC AAC TCG GGC   6408
   Ala Val Glu Ala Ala Lys Lys Leu Lys Ala Lys Gly Tyr Asn Ser Gly
   2000                2005                2010                2015
   TAC TAC TAC AGC GGA GAG GAC CCA TCT AAC CTG AGG GTG GTG ACG TCG   6456
   Tyr Tyr Tyr Ser Gly Glu Asp Pro Ser Asn Leu Arg Val Val Thr Ser
                   2020                2025                2030
   CAG TCC CCA TAC GTG GTG GTA GCA ACC AAC GCA ATA GAA TCG GGC GTT   6504
   Gln Ser Pro Tyr Val Val Val Ala Thr Asn Ala Ile Glu Ser Gly Val
               2035                2040                2045
   ACC CTC CCG GAC CTG GAC GTG GTT GTC GAC ACG GGA CTC AAG TGT GAA   6552
   Thr Leu Pro Asp Leu Asp Val Val Val Asp Thr Gly Leu Lys Cys Glu
           2050                2055                2060
   AAA AGA ATC CGA CTG TCA CCC AAG ATG CCT TTC ATA GTG ACG GGC CTG   6600
   Lys Arg Ile Arg Leu Ser Pro Lys Met Pro Phe Ile Val Thr Gly Leu
       2065                2070                2075
   AAA AGA ATG GCC GTC ACT ATT GGG GAA CAA GCC CAG AGA AGA GGG AGG   6648
   Lys Arg Met Ala Val Thr Ile Gly Glu Gln Ala Gln Arg Arg Gly Arg
   2080                2085                2090                2095
   GTT GGA AGA GTG AAG CCC GGG AGA TAC TAC AGG AGT CAA GAA ACA CCT   6696
   Val Gly Arg Val Lys Pro Gly Arg Tyr Tyr Arg Ser Gln Glu Thr Pro
                   2100                2105                2110
   GTC GGC TCT AAA GAC TAC CAT TAT GAC TTA TTG CAA GCC CAG AGG TAC   6744
   Val Gly Ser Lys Asp Tyr His Tyr Asp Leu Leu Gln Ala Gln Arg Tyr
               2115                2120                2125
   GGC ATA GAA GAT GGG ATA AAT ATC ACC AAA TCC TTC AGA GAG ATG AAC  6792
   Gly Ile Glu Asp Gly Ile Asn Ile Thr Lys Ser Phe Arg Glu Met Asn
           2130                2135                2140
   TAC GAC TGG AGC CTT TAT GAG GAA GAT AGC CTG ATG ATC ACA CAA CTG  6840
   Tyr Asp Trp Ser Leu Tyr Glu Glu Asp Ser Leu Met Ile Thr Gln Leu
       2145                2150                2155
   GAA ATC CTC AAC AAC CTG TTG ATA TCA GAA GAG CTG CCG ATG GCA GTA  6888
   Glu Ile Leu Asn Asn Leu Leu Ile Ser Glu Glu Leu Pro Met Ala Val
   2160                2165                2170                2175
   AAA AAT ATA ATG GCC AGG ACC GAC CAC CCA GAA CCA ATT CAA CTC GCG  6936
   Lys Asn Ile Met Ala Arg Thr Asp His Pro Glu Pro Ile Gln Leu Ala
                   2180                2185                2190
   TAT AAC AGC TAC GAG ACA CAG GTG CCG GTA TTA TTC CCA AAA ATA AGA  6984
   Tyr Asn Ser Tyr Glu Thr Gln Val Pro Val Leu Phe Pro Lys Ile Arg
               2195                2200                2205
   AAT GGA GAG GTG ACT GAT ACT TAC GAT AAT TAC ACC TTC CTC AAT GCA  7032
   Asn Gly Glu Val Thr Asp Thr Tyr Asp Asn Tyr Thr Phe Leu Asn Ala
           2210                2215                2220
   AGA AAA TTG GGA GAT GAC GTA CCC CCC TAC GTG TAT GCT ACA GAG GAT  7080
   Arg Lys Leu Gly Asp Asp Val Pro Pro Tyr Val Tyr Ala Thr Glu Asp
       2225                2230                2235
   GAG GAC TTG GCA GTG GAA CTG TTG GGC CTA GAT TGG CCG GAC CCA GGA  7128
   Glu Asp Leu Ala Val Glu Leu Leu Gly Leu Asp Trp Pro Asp Pro Gly
   2240                2245                2250                2255
   AAC CAA GGC ACC GTG GAA GCT GGC AGA GCA CTA AAA CAG GTG GTT GGT  7176
   Asn Gln Gly Thr Val Glu Ala Gly Arg Ala Leu Lys Gln Val Val Gly
                   2260                2265                2270
   CTA TCA ACA GCA GAG AAC GCC CTG CTA GTC GCC CTG TTC GGC TAC GTG  7224
   Leu Ser Thr Ala Glu Asn Ala Leu Leu Val Ala Leu Phe Gly Tyr Val
               2275                2280                2285
   GGG TAC CAG GCG CTT TCA AAG AGA CAT ATA CCA GTG GTC ACA GAT ATA  7272
   Gly Tyr Gln Ala Leu Ser Lys Arg His Ile Pro Val Val Thr Asp Ile
           2290                2295                2300
   TAT TCA GTA GAA GAT CAC AGG CTA GAG GAC ACT ACG CAC CTA CAG TAT  7320
   Tyr Ser Val Glu Asp His Arg Leu Glu Asp Thr Thr His Leu Gln Tyr
       2305                2310                2315
   GCT CCG AAT GCC ATC AAG ACG GAG GGG AAG GAA ACT GAA TTG AAG GAG  7368
   Ala Pro Asn Ala Ile Lys Thr Glu Gly Lys Glu Thr Glu Leu Lys Glu
   2320                2325                2330                2335
   CTG GCT CAG GGG GAT GTG CAG AGA TGT GTG GAA GCA GTG ACC AAT TAT  7416
   Leu Ala Gln Gly Asp Val Gln Arg Cys Val Glu Ala Val Thr Asn Tyr
                   2340                2345                2350
   GCG AGA GAG GGC ATC CAA TTC ATG AAG TCG CAG GCA CTG AAA GTG AGA   7464
   Ala Arg Glu Gly Ile Gln Phe Met Lys Ser Gln Ala Leu Lys Val Arg
               2355                2360                2365
   GAA ACC CCT ACC TAT AAA GAG ACA ATG AAC ACC GTG GCA GAT TAT GTG   7512
   Glu Thr Pro Thr Tyr Lys Glu Thr Met Asn Thr Val Ala Asp Tyr Val
           2370                2375                2380
   AAA AAG TTT ATT GAG GCA CTG ACG GAT AGC AAG GAA GAC ATC ATT AAA   7560
   Lys Lys Phe Ile Glu Ala Leu Thr Asp Ser Lys Glu Asp Ile Ile Lys
       2385                2390                2395
   TAT GGG CTG TGG GGG GCA CAT ACG GCA TTG TAT AAG AGC ATT GGT GCC   7608
   Tyr Gly Leu Trp Gly Ala His Thr Ala Leu Tyr Lys Ser Ile Gly Ala
   2400                2405                2410                2415
   AGG CTT GGT CAC GAA ACC GCG TTC GCA ACT CTA GTT GTG AAG TGG TTG   7656
   Arg Leu Gly His Glu Thr Ala Phe Ala Thr Leu Val Val Lys Trp Leu
                   2420                2425                2430
   GCA TTT GGG GGG GAG TCA ATA TCA GAC CAC ATA AAG CAA GCG GCC ACA   7704
   Ala Phe Gly Gly Glu Ser Ile Ser Asp His Ile Lys Gln Ala Ala Thr
               2435                2440                2445
   GAC TTG GTC GTT TAT TAC ATT ATT AAC AGA CCT CAA TTC CCA GGA GAC   7752
   Asp Leu Val Val Tyr Tyr Ile Ile Asn Arg Pro Gln Phe Pro Gly Asp
           2450                2455                2460
   ACA GAA ACA CAA CAA GAA GGG AGA AAA TTT GTT GCC AGC CTG CTA GTC   7800
   Thr Glu Thr Gln Gln Glu Gly Arg Lys Phe Val Ala Ser Leu Leu Val
       2465                2470                2475
   TCA GCT CTA GCG ACT TAT ACA TAC AAG AGC TGG AAC TAC AAT AAT CTG   7848
   Ser Ala Leu Ala Thr Tyr Thr Tyr Lys Ser Trp Asn Tyr Asn Asn Leu
   2480                2485                2490                2495
   TCC AAA ATA GTT GAA CCG GCT TTG GCT ACC CTG CCC TAT GCC GCT AAA   7896
   Ser Lys Ile Val Glu Pro Ala Leu Ala Thr Leu Pro Tyr Ala Ala Lys
                   2500                2505                2510
   GCC CTC AAG CTA TTT GCT CCT ACC CGA CTG GAG AGC GTT GTC ATA CTG   7944
   Ala Leu Lys Leu Phe Ala Pro Thr Arg Leu Glu Ser Val Val Ile Leu
               2515                2520                2525
   AGC ACT GCA ATC TAC AAA ACA TAC CTA TCA ATA AGG CGA GGC AAA AGT   7992
   Ser Thr Ala Ile Tyr Lys Thr Tyr Leu Ser Ile Arg Arg Gly Lys Ser
           2530                2535                2540
   GAT GGT CTG CTA GGT ACA GGG GTT AGC GCG GCC ATG GAA ATT ATG TCA   8040
   Asp Gly Leu Leu Gly Thr Gly Val Ser Ala Ala Met Glu Ile Met Ser
       2545                2550                2555
   CAA AAC CCA GTA TCT GTG GGT ATA GCA GTT ATG CTA GGG GTA GGG GCT   8088
   Gln Asn Pro Val Ser Val Gly Ile Ala Val Met Leu Gly Val Gly Ala
   2560                2565                2570                2575
   GTA GCA GCC CAC AAT GCA ATT GAA GCC AGT GAG CAA AAA AGA ACA CTA   8136
   Val Ala Ala His Asn Ala Ile Glu Ala Ser Glu Gln Lys Arg Thr Leu
                       2580                2585                2590
   CTT ATG AAA GTC TTT GTG AAA AAC TTC TTA GAC CAG GCC GCC ACC GAC   8184
   Leu Met Lys Val Phe Val Lys Asn Phe Leu Asp Gln Ala Ala Thr Asp
                   2595                2600                2605
   GAA CTA GTC AAA GAG AGC CCT GAG AAA ATA ATA ATG GCT TTG TTC GAA   8232
   Glu Leu Val Lys Glu Ser Pro Glu Lys Ile Ile Met Ala Leu Phe Glu
               2610                2615                2620
   GCG GTG CAA ACG GTG GGC AAC CCT CTT AGA TTA GTG TAC CAC CTC TAT   8280
   Ala Val Gln Thr Val Gly Asn Pro Leu Arg Leu Val Tyr His Leu Tyr
           2625                2630                2635
   GGA GTT TTC TAT AAA GGG TGG GAA GCA AAA GAG TTG GCC CAA AGA ACA   8328
   Gly Val Phe Tyr Lys Gly Trp Glu Ala Lys Glu Leu Ala Gln Arg Thr
   2640                2645                2650                2655
   GCC GGC AGG AAC CTT TTC ACC TTG ATA ATG TTC GAG GCT GTG GAA CTA   8376
   Ala Gly Arg Asn Leu Phe Thr Leu Ile Met Phe Glu Ala Val Glu Leu
                       2660                2665                2670
   CTG GGA GTA GAC AGT GAG GGA AAA ATT CGC CAG CTA TCG AGC AAT TAC   8424
   Leu Gly Val Asp Ser Glu Gly Lys Ile Arg Gln Leu Ser Ser Asn Tyr
                   2675                2680                2685
   ATA CTA GAG CTC TTG TAT AAG TTC CGC GAC AAT ATC AAG TCT AGT GTG   8472
   Ile Leu Glu Leu Leu Tyr Lys Phe Arg Asp Asn Ile Lys Ser Ser Val
               2690                2695                2700
   AGG GAG ATA GCA ATC AGC TGG GCC CCC GCC CCC TTT AGT TGC GAT TGG   8520
   Arg Glu Ile Ala Ile Ser Trp Ala Pro Ala Pro Phe Ser Cys Asp Trp
           2705                2710                2715
   ACA CCA ACA GAT GAC AGA ATA GGG CTT CCC CAT GAC AAT TAC CTC CGA   8568
   Thr Pro Thr Asp Asp Arg Ile Gly Leu Pro His Asp Asn Tyr Leu Arg
   2720                2725                2730                2735
   GTG GAG ACA AAG TGC CCC TGT GGT TAC AGG ATG AAA GCG GTA AAA AAC   8616
   Val Glu Thr Lys Cys Pro Cys Gly Tyr Arg Met Lys Ala Val Lys Asn
                       2740                2745                2750
   TGC GCT GGG GAG TTG AGA CTT CTG GAG GAA GGG GGT TCA TTC CTC TGC   8664
   Cys Ala Gly Glu Leu Arg Leu Leu Glu Glu Gly Gly Ser Phe Leu Cys
                   2755                2760                2765
   AGA AAT AAA TTC GGT AGA GGC TCA CAA AAC TAC AGG GTG ACA AAA TAC   8712
   Arg Asn Lys Phe Gly Arg Gly Ser Gln Asn Tyr Arg Val Thr Lys Tyr
               2770                2775                2780
   TAT GAT GAC AAT TTA TCA GAA ATA AAA CCA GTG ATA AGA ATG GAA GGA   8760
   Tyr Asp Asp Asn Leu Ser Glu Ile Lys Pro Val Ile Arg Met Glu Gly
           2785                2790                2795
   CAC GTG GAA CTG TAT TAC AAG GGG GCC ACT ATC AAA CTG GAT TTT AAC   8808
   His Val Glu Leu Tyr Tyr Lys Gly Ala Thr Ile Lys Leu Asp Phe Asn
   2800                2805                2810                2815
   AAC AGT AAA ACG GTA CTG GCA ACT GAC AAA TGG GAG GTT GAC CAC TCC   8856
   Asn Ser Lys Thr Val Leu Ala Thr Asp Lys Trp Glu Val Asp His Ser
                   2820                2825                2830
   ACC CTG GTT AGG GCA CTC AAG AGG TAC ACA GGG GCT GGA TAT CGA GGG   8904
   Thr Leu Val Arg Ala Leu Lys Arg Tyr Thr Gly Ala Gly Tyr Arg Gly
               2835                2840                2845
   GCG TAT TTG GGT GAG AAA CCT AAC CAT AAA CAT CTG ATA CAG AGA GAC   8952
   Ala Tyr Leu Gly Glu Lys Pro Asn His Lys His Leu Ile Gln Arg Asp
           2850                2855                2860
   TGT GCA ACG ATT ACC AAA GAC AAG GTC TGC TTC ATC AAA ATG AAG AGA   9000
   Cys Ala Thr Ile Thr Lys Asp Lys Val Cys Phe Ile Lys Met Lys Arg
       2865                2870                2875
   GGG TGT GCG TTC ACT TAT GAC CTA TCC CTC CAC AAC CTT ACC CGG CTA   9048
   Gly Cys Ala Phe Thr Tyr Asp Leu Ser Leu His Asn Leu Thr Arg Leu
   2880                2885                2890                2895
   ATC GAA TTG GTA CAC AAG AAT AAC CTG GAA GAT AGA GAA ATC CCT GCT   9096
   Ile Glu Leu Val His Lys Asn Asn Leu Glu Asp Arg Glu Ile Pro Ala
                   2900                2905                2910
   GTG ACG GTT ACA ACC TGG CTG GCC TAC ACA TTT GTG AAT GAA GAC ATA   9144
   Val Thr Val Thr Thr Trp Leu Ala Tyr Thr Phe Val Asn Glu Asp Ile
               2915                2920                2925
   GGG ACC ATA AAA CCA ACT TTT GGG GAA AAG GTG ACA CCG GAG AAA CAG   9192
   Gly Thr Ile Lys Pro Thr Phe Gly Glu Lys Val Thr Pro Glu Lys Gln
           2930                2935                2940
   GAG GAG GTA GTC TTG CAG CCT GCT GTG GTG GTG GAC ACA ACA GAT GTA   9240
   Glu Glu Val Val Leu Gln Pro Ala Val Val Val Asp Thr Thr Asp Val
       2945                2950                2955
   GCC GTG ACC GTG GTA GGG GAA ACC TCT ACT ATG ACT ACA GGG GAG ACC   9288
   Ala Val Thr Val Val Gly Glu Thr Ser Thr Met Thr Thr Gly Glu Thr
   2960                2965                2970                2975
   CCG ACA ACA TTT ACC AGC TTA GGT TCG GAC TCG AAG GTC CGA CAA GTC   9336
   Pro Thr Thr Phe Thr Ser Leu Gly Ser Asp Ser Lys Val Arg Gln Val
                   2980                2985                2990
   CTG AAG CTG GGC GTG GAC GAT GGT CAA TAC CCC GGG CCT AAT CAG CAG   9384
   Leu Lys Leu Gly Val Asp Asp Gly Gln Tyr Pro Gly Pro Asn Gln Gln
               2995                3000                3005
   AGA GCA AGC CTG CTC GAA GCT ATA CAA GGT GTG GAT GAA AGG CCC TCG   9432
   Arg Ala Ser Leu Leu Glu Ala Ile Gln Gly Val Asp Glu Arg Pro Ser
           3010                3015                3020
   GTA CTG ATA CTG GGG TCT GAT AAG GCC ACC TCC AAT AGG GTC AAG ACC   9480
   Val Leu Ile Leu Gly Ser Asp Lys Ala Thr Ser Asn Arg Val Lys Thr
       3025                3030                3035
   GCA AAG AAT GTG AAG ATA TAT AGG AGC AGG GAC CCC CTG GAA CTG AGA   9528
   Ala Lys Asn Val Lys Ile Tyr Arg Ser Arg Asp Pro Leu Glu Leu Arg
   3040                3045                3050                3055
   GAA ATG ATG AAA AGG GGA AAA ATC CTA GTC GTA GCC TTG TCT AGA GTC   9576
   Glu Met Met Lys Arg Gly Lys Ile Leu Val Val Ala Leu Ser Arg Val
                   3060                3065                3070
   GAT ACC GCT CTG CTG AAA TTC GTT GAT TAC AAA GGC ACC TTC CTG ACC   9624
   Asp Thr Ala Leu Leu Lys Phe Val Asp Tyr Lys Gly Thr Phe Leu Thr
               3075                3080                3085
   AGA GAG ACC CTA GAG GCA TTA AGT CTG GGT AAG CCT AAG AAA AGA GAC   9672
   Arg Glu Thr Leu Glu Ala Leu Ser Leu Gly Lys Pro Lys Lys Arg Asp
           3090                3095                3100
   ATA ACT AAA GCA GAA GCA CAA TGG CTG CTG CGC CTC GAA GAC CAA ATA   9720
   Ile Thr Lys Ala Glu Ala Gln Trp Leu Leu Arg Leu Glu Asp Gln Ile
       3105                3110                3115
   GAA GAG CTG CCT GAC TGG TTC GCA GCC AAG GAA CCC ATA TTT CTA GAA   9768
   Glu Glu Leu Pro Asp Trp Phe Ala Ala Lys Glu Pro Ile Phe Leu Glu
   3120                3125                3130                3135
   GCC AAC ATT AAA CGT GAC AAG TAT CAC CTG GTA GGG GAC ATA GCC ACT   9816
   Ala Asn Ile Lys Arg Asp Lys Tyr His Leu Val Gly Asp Ile Ala Thr
                   3140                3145                3150
   ATT AAA GAA AAA GCC AAA CAA CTG GGG GCA ACA GAC TCC ACA AAG ATA   9864
   Ile Lys Glu Lys Ala Lys Gln Leu Gly Ala Thr Asp Ser Thr Lys Ile
               3155                3160                3165
   TCA AAG GAG GTT GGC GCG AAA GTG TAT TCT ATG AAG CTG AGT AAC TGG   9912
   Ser Lys Glu Val Gly Ala Lys Val Tyr Ser Met Lys Leu Ser Asn Trp
           3170                3175                3180
   GTG ATA CAA GAA GAG AAT AAA CAA GGC AGC CTT GCC CCC CTG TTT GAA   9960
   Val Ile Gln Glu Glu Asn Lys Gln Gly Ser Leu Ala Pro Leu Phe Glu
       3185                3190                3195
   GAG CTC CTG CAA CAG TGC CCA CCC GGG GGC CAG AAC AAA ACC ACA CAT 10008
   Glu Leu Leu Gln Gln Cys Pro Pro Gly Gly Gln Asn Lys Thr Thr His
   3200                3205                3210                3215
   ATG GTC TCA GCC TAC CAA CTA GCT CAA GGG AAT TGG GTG CCA GTT AGT 10056
   Met Val Ser Ala Tyr Gln Leu Ala Gln Gly Asn Trp Val Pro Val Ser
                   3220                3225                3230
   TGC CAC GTG TTC ATG GGG ACC ATA CCC GCC AGA AGA ACC AAG ACT CAT 10104
   Cys His Val Phe Met Gly Thr Ile Pro Ala Arg Arg Thr Lys Thr His
               3235                3240                3245
   CCT TAT GAG GCA TAC GTT AAG CTA AGG GAG TTG GTA GAT GAA CAT AAG 10152
   Pro Tyr Glu Ala Tyr Val Lys Leu Arg Glu Leu Val Asp Glu His Lys
               3250                3255                3260
   ATG AAG GCA TTA TGT GGC GGA TCA GGC CTA AGT AAG CAC AAC GAA TGG 10200
   Met Lys Ala Leu Cys Gly Gly Ser Gly Leu Ser Lys His Asn Glu Trp
         3265                3270                3275
   GTA ATT GGC AAG GTC AAG TAT CAA GGA AAC CTG AGG ACC AAA CAC ATG 10248
   Val Ile Gly Lys Val Lys Tyr Gln Gly Asn Leu Arg Thr Lys His Met
   3280                3285                3290                3295
   TTG AAC CCC GGA AAG GTG GCG GAG CAA CTG CAC AGA GAA GGG TAC AGG 10296
   Leu Asn Pro Gly Lys Val Ala Glu Gln Leu His Arg Glu Gly Tyr Arg
                     3300                3305                3310
   CAC AAT GTG TAT AAT AAG ACA ATA GGT TCA GTG ATG ACA GCA ACT GGT 10344
   His Asn Val Tyr Asn Lys Thr Ile Gly Ser Val Met Thr Ala Thr Gly
                 3315                3320                3325
   ATC AGG CTG GAG AAG TTA CCT GTG GTT AGG GCC CAA ACA GAC ACA ACC 10392
   Ile Arg Leu Glu Lys Leu Pro Val Val Arg Ala Gln Thr Asp Thr Thr
               3330                3335                3340
   AAC TTC CAC CAA GCA ATA AGG GAT AAA ATA GAC AAG GAG GAG AAC CTA 10440
   Asn Phe His Gln Ala Ile Arg Asp Lys Ile Asp Lys Glu Glu Asn Leu
         3345                3350                3355
   CAG ACC CCT GGC TTG CAT AAG AAG TTA ATG GAA GTC TTC AAT GCA TTA 10488
   Gln Thr Pro Gly Leu His Lys Lys Leu Met Glu Val Phe Asn Ala Leu
   3360                3365                3370                3375
   AAA AGA CCC GAG CTT GAG GCC TCT TAT GAC GCT GTG GAT TGG GAG GAA 10536
   Lys Arg Pro Glu Leu Glu Ala Ser Tyr Asp Ala Val Asp Trp Glu Glu
                     3380                3385                3390
   TTG GAG AGA GGA ATA AAT AGG AAG GGT GCT GCT GGT TTC TTC GAA CGC 10584
   Leu Glu Arg Gly Ile Asn Arg Lys Gly Ala Ala Gly Phe Phe Glu Arg
                 3395                3400                3405
   AAG AAC ATA GGA GAG GTT TTG GAT TCG GAA AAA AAT AAA GTC GAA GAG 10632
   Lys Asn Ile Gly Glu Val Leu Asp Ser Glu Lys Asn Lys Val Glu Glu
               3410                3415                3420
   GTT ATT GAC AGT TTG AAA AAA GGT AGG AAT ATC AGA TAC TAC GAA ACT 10680
   Val Ile Asp Ser Leu Lys Lys Gly Arg Asn Ile Arg Tyr Tyr Glu Thr
         3425                3430                3435
   GCA ATC CCG AAA AAC GAG AAG AGG GAT GTC AAT GAT GAC TGG ACC GCT 10728
   Ala Ile Pro Lys Asn Glu Lys Arg Asp Val Asn Asp Asp Trp Thr Ala
   3440                3445                3450                3455
   GGT GAC TTC GTA GAT GAG AAG AAG CCA AGA GTG ATA CAA TAC CCT GAG 10776
   Gly Asp Phe Val Asp Glu Lys Lys Pro Arg Val Ile Gln Tyr Pro Glu
                     3460                3465                3470
   GCT AAA ACT AGG TTG GCT ATT ACT AAG GTA ATG TAC AAG TGG GTC AAA 10824
   Ala Lys Thr Arg Leu Ala Ile Thr Lys Val Met Tyr Lys Trp Val Lys
               3475                3480                3485
   CAG AAG CCA GTT GTC ATA CCG GGT TAT GAA GGT AAG ACA CCC CTG TTT 10872
   Gln Lys Pro Val Val Ile Pro Gly Tyr Glu Gly Lys Thr Pro Leu Phe
           3490                3495                3500
   CAA ATT TTT GAC AAA GTG AAG AAA GAA TGG GAT CAA TTC CAA AAC CCT 10920
   Gln Ile Phe Asp Lys Val Lys Lys Glu Trp Asp Gln Phe Gln Asn Pro
       3505                3510                3515
   GTG GCA GTT AGC TTT GAT ACC AAA GCG TGG GAT ACC CAG GTA ACC ACA 10968
   Val Ala Val Ser Phe Asp Thr Lys Ala Trp Asp Thr Gln Val Thr Thr
   3520                3525                3530                3535
   AGG GAT TTG GAG CTA ATA AGG GAT ATA CAG AAG TTC TAT TTT AAA AAG 11016
   Arg Asp Leu Glu Leu Ile Arg Asp Ile Gln Lys Phe Tyr Phe Lys Lys
                   3540                3545                3550
   AAA TGG CAC AAA TTC ATT GAC ACC CTA ACC AAG CAC ATG TCA GAA GTA 11064
   Lys Trp His Lys Phe Ile Asp Thr Leu Thr Lys His Met Ser Glu Val
               3555                3560                3565
   CCC GTA ATC AGT GCC GAC GGG GAG GTA TAC ATA AGG AAA GGT CAG AGA 11112
   Pro Val Ile Ser Ala Asp Gly Glu Val Tyr Ile Arg Lys Gly Gln Arg
           3570                3575                3580
   GGC AGT GGG CAA CCT GAC ACG AGC GCA GGC AAC AGC ATG TTG AAT GTG 11160
   Gly Ser Gly Gln Pro Asp Thr Ser Ala Gly Asn Ser Met Leu Asn Val
       3585                3590                3595
   TTG ACA ATG GTG TAT GCC TTC TGC GAG GCC ACG GGG GTA CCC TAC AAG 11208
   Leu Thr Met Val Tyr Ala Phe Cys Glu Ala Thr Gly Val Pro Tyr Lys
   3600                3605                3610                3615
   AGT TTT GAC AGA GTG GCA AAG ATC CAT GTC TGC GGG GAT GAT GGT TTC 11256
   Ser Phe Asp Arg Val Ala Lys Ile His Val Cys Gly Asp Asp Gly Phe
                   3620                3625                3630
   CTG ATT ACC GAA AGA GCT CTC GGT GAG AAA TTT GCG AGT AAA GGA GTC 11304
   Leu Ile Thr Glu Arg Ala Leu Gly Glu Lys Phe Ala Ser Lys Gly Val
               3635                3640                3645
   CAG ATC CTA TAC GAA GCT GGG AAG CCT CAA AAG ATC ACT GAA GGG GAC 11352
   Gln Ile Leu Tyr Glu Ala Gly Lys Pro Gln Lys Ile Thr Glu Gly Asp
           3650                3655                3660
   AAG ATG AAA GTA GCC TAT CAG TTT GAT GAT ATC GAG TTC TGC TCC CAT 11400
   Lys Met Lys Val Ala Tyr Gln Phe Asp Asp Ile Glu Phe Cys Ser His
       3665                3670                3675
   ACA CCA GTA CAA GTG AGG TGG TCA GAC AAT ACT TCC AGC TAC ATG CCG 11448
   Thr Pro Val Gln Val Arg Trp Ser Asp Asn Thr Ser Ser Tyr Met Pro
   3680                3685                3690                3695
   GGA AGG AAC ACG ACT ACA ATC CTG GCT AAA ATG GCT ACA AGG TTG GAT 11496
   Gly Arg Asn Thr Thr Thr Ile Leu Ala Lys Met Ala Thr Arg Leu Asp
                   3700                3705                3710
   TCC AGT GGT GAG AGG GGT ACT ATA GCA TAT GAG AAG GCA GTG GCG TTC 11544
   Ser Ser Gly Glu Arg Gly Thr Ile Ala Tyr Glu Lys Ala Val Ala Phe
               3715                3720                3725
   AGC TTT TTG TTG ATG TAC TCC TGG AAC CCA CTG ATC AGA AGG ATA TGC 11592
   Ser Phe Leu Leu Met Tyr Ser Trp Asn Pro Leu Ile Arg Arg Ile Cys
           3730                3735                3740
   TTA CTG GTG TTG TCA ACT GAG TTG CAA GTG AGA CCA GGG AAG TCA ACC 11640
   Leu Leu Val Leu Ser Thr Glu Leu Gln Val Arg Pro Gly Lys Ser Thr
       3745                3750                3755
   ACC TAT TAC TAT GAA GGG GAC CCA ATA TCC GCT TAC AAG GAA GTC ATT 11688
   Thr Tyr Tyr Tyr Glu Gly Asp Pro Ile Ser Ala Tyr Lys Glu Val Ile
   3760                3765                3770                3775
   GGC CAC AAT CTC TTT GAC CTT AAA AGA ACA AGC TTC GAA AAG CTA GCA 11736
   Gly His Asn Leu Phe Asp Leu Lys Arg Thr Ser Phe Glu Lys Leu Ala
                   3780                3785                3790
   AAG TTA AAT CTC AGC ATG TCC ACG CTC GGG GTG TGG ACT AGA CAC ACT 11784
   Lys Leu Asn Leu Ser Met Ser Thr Leu Gly Val Trp Thr Arg His Thr
               3795                3800                3805
   AGC AAG AGA TTA CTA CAA GAT TGT GTC AAT GTT GGC ACC AAA GAG GGC 11832
   Ser Lys Arg Leu Leu Gln Asp Cys Val Asn Val Gly Thr Lys Glu Gly
           3810                3815                3820
   AAC TGG CTG GTC AAT GCA GAC AGA CTA GTG AGT AGT AAG ACA GGA AAC 11880
   Asn Trp Leu Val Asn Ala Asp Arg Leu Val Ser Ser Lys Thr Gly Asn
       3825                3830                3835
   AGG TAT ATA CCT GGA GAG GGC CAC ACC CTA CAA GGG AAA CAT TAT GAA 11928
   Arg Tyr Ile Pro Gly Glu Gly His Thr Leu Gln Gly Lys His Tyr Glu
   3840                3845                3850                3855
   GAA CTG ATA CTG GCA AGG AAA CCG ATC GGT AAC TTT GAA GGG ACC GAT 11976
   Glu Leu Ile Leu Ala Arg Lys Pro Ile Gly Asn Phe Glu Gly Thr Asp
                   3860                3865                3870
   AGG TAT AAC TTG GGG CCA ATA GTC AAT GTA GTG TTG AGG AGA CTA AAA 12024
   Arg Tyr Asn Leu Gly Pro Ile Val Asn Val Val Leu Arg Arg Leu Lys
               3875                3880                3885
   ATT ATG ATG ATG GCC CTG ATA GGA AGG GGG GTG TGAGCATGGT TGGCCCTTGA 12077
   Ile Met Met Met Ala Leu Ile Gly Arg Gly Val
           3890                3895
   TCGGGCCCTA TCAGTAGAAC CCTATTGTAA ATAACATTAA CTTATTAATT ATTTAGATAC 12137
   TATTATTTAT TTATTTATTT ATTTATTGAA TGAGCAAGTA CTGGTACAAA CTACCTCATG 12197
   TTACCACACT ACACTCATTT TAACAGCACT TTAGCTGGAG GGAAAACCCT GACGTCCACA 12257
   GTTGGACTAA GGTAATTTCC TAACGGC                                    12284
(2) INFORMATION FOR SEQ ID NO:2:
   (i) SEQUENCE CHARACTERISTICS:
      (A) LENGTH: 3898 amino acids
      (B) TYPE: amino acid
      (D) TOPOLOGY: linear
   (ii) MOLECULE TYPE: protein
   (xi) SEQUENCE DESCRIPTION: SEQ ID NO:2:
   Met Glu Leu Asn His Phe Glu Leu Leu Tyr Lys Thr Ser Lys Gln Lys
    1               5                  10                  15
   Pro Val Gly Val Glu Glu Pro Val Tyr Asp Thr Ala Gly Arg Pro Leu
               20                  25                  30
   Phe Gly Asn Pro Ser Glu Val His Pro Gln Ser Thr Leu Lys Leu Pro
           35                  40                  45
   His Asp Arg Gly Arg Gly Asp Ile Arg Thr Thr Leu Arg Asp Leu Pro
       50                  55                  60
   Arg Lys Gly Asp Cys Arg Ser Gly Asn His Leu Gly Pro Val Ser Gly
   65                  70                  75                  80
   Ile Tyr Ile Lys Pro Gly Pro Val Tyr Tyr Gln Asp Tyr Thr Gly Pro
                   85                  90                  95
   Val Tyr His Arg Ala Pro Leu Glu Phe Phe Asp Glu Ala Gln Phe Cys
               100                 105                 110
   Glu Val Thr Lys Arg Ile Gly Arg Val Thr Gly Ser Asp Gly Lys Leu
           115                 120                 125
   Tyr His Ile Tyr Val Cys Val Asp Gly Cys Ile Leu Leu Lys Leu Ala
       130                 135                 140
   Lys Arg Gly Thr Pro Arg Thr Leu Lys Trp Ile Arg Asn Phe Thr Asn
   145                 150                 155                 160
   Cys Pro Leu Trp Val Thr Ser Cys Ser Asp Asp Gly Ala Ser Gly Ser
                   165                 170                 175
   Lys Asp Lys Lys Pro Asp Arg Met Asn Lys Gly Lys Leu Lys Ile Ala
               180                 185                 190
   Pro Arg Glu His Glu Lys Asp Ser Lys Thr Lys Pro Pro Asp Ala Thr
           195                 200                 205
   Ile Val Val Glu Gly Val Lys Tyr Gln Ile Lys Lys Lys Gly Lys Val
       210                 215                 220
   Lys Gly Lys Asn Thr Gln Asp Gly Leu Tyr His Asn Lys Asn Lys Pro
   225                 230                 235                     240
   Pro Glu Ser Arg Lys Lys Leu Glu Lys Ala Leu Leu Ala Trp Ala Val
                   245                 250                 255
   Ile Thr Ile Leu Leu Tyr Gln Pro Val Ala Ala Glu Asn Ile Thr Gln
               260                 265                 270
   Trp Asn Leu Ser Asp Asn Gly Thr Asn Gly Ile Gln Arg Ala Met Tyr
           275                 280                 285
   Leu Arg Gly Val Asn Arg Ser Leu His Gly Ile Trp Pro Glu Lys Ile
       290                 295                 300
   Cys Lys Gly Val Pro Thr His Leu Ala Thr Asp Thr Glu Leu Lys Glu
   305                 310                 315                     320
   Ile Arg Gly Met Met Asp Ala Ser Glu Arg Thr Asn Tyr Thr Cys Cys
                   325                 330                 335
   Arg Leu Gln Arg His Glu Trp Asn Lys His Gly Trp Cys Asn Trp Tyr
               340                 345                 350
   Asn Ile Asp Pro Trp Ile Gln Leu Met Asn Arg Thr Gln Thr Asn Leu
           355                 360                 365
   Thr Glu Gly Pro Pro Asp Lys Glu Cys Ala Val Thr Cys Arg Tyr Asp
       370                 375                 380
   Lys Asn Thr Asp Val Asn Val Val Thr Gln Ala Arg Asn Arg Pro Thr
   385                 390                 395                     400
   Thr Leu Thr Gly Cys Lys Lys Gly Lys Asn Phe Ser Phe Ala Gly Thr
                   405                 410                 415
   Val Ile Glu Gly Pro Cys Asn Phe Asn Val Ser Val Glu Asp Ile Leu
               420                 425                 430
   Tyr Gly Asp His Glu Cys Gly Ser Leu Leu Gln Asp Thr Ala Leu Tyr
           435                 440                 445
   Leu Leu Asp Gly Met Thr Asn Thr Ile Glu Asn Ala Arg Gln Gly Ala
       450                 455                 460
   Ala Arg Val Thr Ser Trp Leu Gly Arg Gln Leu Ser Thr Ala Gly Lys
   465                 470                 475                     480
   Lys Leu Glu Arg Arg Ser Lys Thr Trp Phe Gly Ala Tyr Ala Leu Ser
                   485                 490                 495
   Pro Tyr Cys Asn Val Thr Arg Lys Ile Gly Tyr Ile Trp Tyr Thr Asn
               500                 505                 510
   Asn Cys Thr Pro Ala Cys Leu Pro Lys Asn Thr Lys Ile Ile Gly Pro
           515                 520                 525
   Gly Lys Phe Asp Thr Asn Ala Glu Asp Gly Lys Ile Leu His Glu Met
       530                 535                 540
   Gly Gly His Leu Ser Glu Phe Leu Leu Leu Ser Leu Val Ile Leu Ser
   545                 550                 555                 560
   Asp Phe Ala Pro Glu Thr Ala Ser Thr Leu Tyr Leu Ile Leu His Tyr
                   565                 570                 575
   Ala Ile Pro Gln Ser His Glu Glu Pro Glu Gly Cys Asp Thr Asn Gln
               580                 585                 590
   Leu Asn Leu Thr Val Lys Leu Arg Thr Glu Asp Val Val Pro Ser Ser
           595                 600                 605
   Val Trp Asn Ile Gly Lys Tyr Val Cys Val Arg Pro Asp Trp Trp Pro
       610                 615                 620
   Tyr Glu Thr Lys Val Ala Leu Leu Phe Glu Glu Ala Gly Gln Val Ile
   625                 630                 635                 640
   Lys Leu Val Leu Arg Ala Leu Arg Asp Leu Thr Arg Val Trp Asn Ser
                   645                 650                 655
   Ala Ser Thr Thr Ala Phe Leu Ile Cys Leu Ile Lys Val Leu Arg Gly
               660                 665                 670
   Gln Val Val Gln Gly Ile Ile Trp Leu Leu Leu Val Thr Gly Ala Gln
           675                 680                 685
   Gly Arg Leu Ala Cys Lys Glu Asp Tyr Arg Tyr Ala Ile Ser Ser Thr
       690                 695                 700
   Asn Glu Ile Gly Leu Leu Gly Ala Glu Gly Leu Thr Thr Thr Trp Lys
   705                 710                 715                 720
   Glu Tyr Ser His Gly Leu Gln Leu Asp Asp Gly Thr Val Lys Ala Val
                   725                 730                 735
   Cys Thr Ala Gly Ser Phe Lys Val Thr Ala Leu Asn Val Val Ser Arg
               740                 745                 750
   Arg Tyr Leu Ala Ser Leu His Lys Arg Ala Leu Pro Thr Ser Val Thr
           755                 760                 765
   Phe Glu Leu Leu Phe Asp Gly Thr Asn Pro Ala Ile Glu Glu Met Asp
       770                 775                 780
   Asp Asp Phe Gly Phe Gly Leu Cys Pro Phe Asp Thr Ser Pro Val Ile
   785 790 795 800
   Lys Gly Lys Tyr Asn Thr Thr Leu Leu Asn Gly Ser Ala Phe Tyr Leu
   805 810 815
   Val Cys Pro Ile Gly Trp Thr Gly Val Val Glu Cys Thr Ala Val Ser
   820 825 830
   Pro Thr Thr Leu Arg Thr Glu Val Val Lys Thr Phe Arg Arg Asp Lys
   835 840 845
   Pro Phe Pro His Arg Val Asp Cys Val Thr Thr Ile Val Glu Lys Glu
   850 855 860
   Asp Leu Phe His Cys Lys Leu Gly Gly Asn Trp Thr Cys Val Lys Gly
   865 870 875 880
   Asp Pro Val Thr Tyr Lys Gly Gly Gln Val Lys Gln Cys Arg Trp Cys
   885 890 895
   Gly Phe Glu Phe Lys Glu Pro Tyr Gly Leu Pro His Tyr Pro Ile Gly
   900 905 910
   Lys Cys Ile Leu Thr Asn Glu Thr Gly Tyr Arg Val Val Asp Ser Thr
   915 920 925
   Asp Cys Asn Arg Asp Gly Val Val Ile Ser Thr Glu Gly Glu His Glu
   930 935 940
   Cys Leu Ile Gly Asn Thr Thr Val Lys Val His Ala Leu Asp Glu Arg
   945 950 955 960
   Leu Gly Pro Met Pro Cys Arg Pro Lys Glu Ile Val Ser Ser Glu Gly
   965 970 975
   Pro Val Arg Lys Thr Ser Cys Thr Phe Asn Tyr Thr Lys Thr Leu Arg
   980 985 990
   Asn Lys Tyr Tyr Glu Pro Arg Asp Ser Tyr Phe Gln Gln Tyr Met Leu
   995 1000 1005
   Lys Gly Glu Tyr Gln Tyr Trp Phe Asn Leu Asp Val Thr Asp His His
   1010 1015 1020
   Thr Asp Tyr Phe Ala Glu Phe Val Val Leu Val Val Val Ala Leu Leu
   1025 1030 1035 1040
   Gly Gly Arg Tyr Val Leu Trp Leu Ile Val Thr Tyr Ile Ile Leu Thr
   1045 1050 1055
   Glu Gln Leu Ala Ala Gly Leu Gln Leu Gly Gln Gly Glu Val Val Leu
   1060 1065 1070
   Ile Gly Asn Leu Ile Thr His Thr Asp Asn Glu Val Val Val Tyr Phe
   1075 1080 1085
   Leu Leu Leu Tyr Leu Val Ile Arg Asp Glu Pro Ile Lys Lys Trp Ile
   1090 1095 1100
   Leu Leu Leu Phe His Ala Met Thr Asn Asn Pro Val Lys Thr Ile Thr
   1105 1110 1115 1120
   Val Ala Leu Leu Met Ile Ser Gly Val Ala Lys Gly Gly Lys Ile Asp
   1125 1130 1135
   Gly Gly Trp Gln Arg Gln Pro Val Thr Ser Phe Asp Ile Gln Leu Ala
   1140 1145 1150
   Leu Ala Val Val Val Val Val Val Met Leu Leu Ala Lys Arg Asp Pro
   1155 1160 1165
   Thr Thr Phe Pro Leu Val Ile Thr Val Ala Thr Leu Arg Thr Ala Lys
   1170 1175 1180
   Ile Thr Asn Gly Phe Ser Thr Asp Leu Val Ile Ala Thr Val Ser Ala
   1185 1190 1195 1200
   Ala Leu Leu Thr Trp Thr Tyr Ile Ser Asp Tyr Tyr Lys Tyr Lys Thr
   1205 1210 1215
   Trp Leu Gln Tyr Leu Val Ser Thr Val Thr Gly Ile Phe Leu Ile Arg
   1220 1225 1230
   Val Leu Lys Gly Ile Gly Glu Leu Asp Leu His Ala Pro Thr Leu Pro
   1235 1240 1245
   Ser His Arg Pro Leu Phe Tyr Ile Leu Val Tyr Leu Ile Ser Thr Ala
   1250 1255 1260
   Val Val Thr Arg Trp Asn Leu Asp Val Ala Gly Leu Leu Leu Gln Cys
   1265 1270 1275 1280
   Val Pro Thr Leu Leu Met Val Phe Thr Met Trp Ala Asp Ile Leu Thr
   1285 1290 1295
   Leu Ile Leu Ile Leu Pro Thr Tyr Glu Leu Thr Lys Leu Tyr Tyr Leu
   1300 1305 1310
   Lys Glu Val Lys Ile Gly Ala Glu Arg Gly Trp Leu Trp Lys Thr Asn
   1315 1320 1325
   Tyr Lys Arg Val Asn Asp Ile Tyr Glu Val Asp Gln Thr Ser Glu Gly
   1330 1335 1340
   Val Tyr Leu Phe Pro Ser Lys Gln Arg Thr Ser Ala Ile Thr Ser Thr
   1345 1350 1355 1360
   Met Leu Pro Leu Ile Lys Ala Ile Leu Ile Ser Cys Ile Ser Asn Lys
   1365 1370 1375
   Trp Gln Leu Ile Tyr Leu Leu Tyr Leu Ile Phe Glu Val Ser Tyr Tyr
   1380 1385 1390
   Leu His Lys Lys Val Ile Asp Glu Ile Ala Gly Gly Thr Asn Phe Val
   1395 1400 1405
   Ser Arg Leu Val Ala Ala Leu Ile Glu Val Asn Trp Ala Phe Asp Asn
   1410 1415 1420
   Glu Glu Val Lys Gly Leu Lys Lys Phe Phe Leu Leu Ser Ser Arg Val
   1425 1430 1435 1440
   Lys Glu Leu Ile Ile Lys His Lys Val Arg Asn Glu Val Val Val Arg
   1445 1450 1455
   Trp Phe Gly Asp Glu Glu Ile Tyr Gly Met Pro Lys Leu Ile Gly Leu
   1460 1465 1470
   Val Lys Ala Ala Thr Leu Ser Arg Asn Lys His Cys Met Leu Cys Thr
   1475 1480 1485
   Val Cys Glu Asp Arg Asp Trp Arg Gly Glu Thr Cys Pro Lys Cys Gly
   1490 1495 1500
   Arg Phe Gly Pro Pro Val Val Cys Gly Met Thr Leu Ala Asp Phe Glu
   1505 1510 1515 1520
   Glu Lys His Tyr Lys Arg Ile Phe Ile Arg Glu Asp Gln Ser Gly Gly
   1525 1530 1535
   Pro Leu Arg Glu Glu His Ala Gly Tyr Leu Gln Tyr Lys Ala Arg Gly
   1540 1545 1550
   Gln Leu Phe Leu Arg Asn Leu Pro Val Leu Ala Thr Lys Val Lys Met
   1555 1560 1565
   Leu Leu Val Gly Asn Leu Gly Thr Glu Ile Gly Asp Leu Glu His Leu
   1570 1575 1580
   Gly Trp Val Leu Arg Gly Pro Ala Val Cys Lys Lys Val Thr Glu His
   1585 1590 1595 1600
   Glu Arg Cys Thr Thr Ser Ile Met Asp Lys Leu Thr Ala Phe Phe Gly
   1605 1610 1615
   Val Met Pro Arg Gly Thr Thr Pro Arg Ala Pro Val Arg Phe Pro Thr
   1620 1625 1630
   Ser Leu Leu Lys Ile Arg Arg Gly Leu Glu Thr Gly Trp Ala Tyr Thr
   1635 1640 1645
   His Gln Gly Gly Ile Ser Ser Val Asp His Val Thr Cys Gly Lys Asp
   1650 1655 1660
   Leu Leu Val Cys Asp Thr Met Gly Arg Thr Arg Val Val Cys Gln Ser
   1665 1670 1675 1680
   Asn Asn Lys Met Thr Asp Glu Ser Glu Tyr Gly Val Lys Thr Asp Ser
   1685 1690 1695
   Gly Cys Pro Glu Gly Ala Arg Cys Tyr Val Phe Asn Pro Glu Ala Val
   1700 1705 1710
   Asn Ile Ser Gly Thr Lys Gly Ala Met Val His Leu Gln Lys Thr Gly
   1715 1720 1725
   Gly Glu Phe Thr Cys Val Thr Ala Ser Gly Thr Pro Ala Phe Phe Asp
   1730 1735 1740
   Leu Lys Asn Leu Lys Gly Trp Ser Gly Leu Pro Ile Phe Glu Ala Ser
   1745 1750 1755 1760
   Ser Gly Arg Val Val Gly Arg Val Lys Val Gly Lys Asn Glu Asp Ser
   1765 1770 1775
   Lys Pro Thr Lys Leu Met Ser Gly Ile Gln Thr Val Ser Lys Ser Ala
   1780 1785 1790
   Thr Asp Leu Thr Glu Met Val Lys Lys Ile Thr Thr Met Asn Arg Gly
   1795 1800 1805
   Glu Phe Arg Gln Ile Thr Leu Ala Thr Gly Ala Gly Lys Thr Thr Glu
   1810 1815 1820
   Leu Pro Arg Ser Val Ile Glu Glu Ile Gly Arg His Lys Arg Val Leu
   1825 1830 1835 1840
   Val Leu Ile Pro Leu Arg Ala Ala Ala Glu Ser Val Tyr Gln Tyr Met
   1845 1850 1855
   Arg Gln Lys His Pro Ser Ile Ala Phe Asn Leu Arg Ile Gly Glu Met
   1860 1865 1870
   Lys Glu Gly Asp Met Ala Thr Gly Ile Thr Tyr Ala Ser Tyr Gly Tyr
   1875 1880 1885
   Phe Cys Gln Met Ser Gln Pro Lys Leu Arg Ala Ala Met Val Glu Tyr
   1890 1895 1900
   Ser Phe Ile Phe Leu Asp Glu Tyr His Cys Ala Thr Pro Glu Gln Leu
   1905 1910 1915 1920
   Ala Ile Met Gly Lys Ile His Arg Phe Ser Glu Asn Leu Arg Val Val
   1925 1930 1935
   Ala Met Thr Ala Thr Pro Ala Gly Thr Val Thr Thr Thr Gly Gln Lys
   1940 1945 1950
   His Pro Ile Glu Glu Phe Ile Ala Pro Glu Val Met Lys Gly Glu Asp
   1955 1960 1965
   Leu Gly Ser Glu Tyr Leu Asp Ile Ala Gly Leu Lys Ile Pro Val Glu
   1970 1975 1980
   Glu Met Lys Asn Asn Met Leu Val Phe Val Pro Thr Arg Asn Met Ala
   1985 1990 1995 2000
   Val Glu Ala Ala Lys Lys Leu Lys Ala Lys Gly Tyr Asn Ser Gly Tyr
   2005 2010 2015
   Tyr Tyr Ser Gly Glu Asp Pro Ser Asn Leu Arg Val Val Thr Ser Gln
   2020 2025 2030
   Ser Pro Tyr Val Val Val Ala Thr Asn Ala Ile Glu Ser Gly Val Thr
   2035 2040 2045
   Leu Pro Asp Leu Asp Val Val Val Asp Thr Gly Leu Lys Cys Glu Lys
   2050 2055 2060
   Arg Ile Arg Leu Ser Pro Lys Met Pro Phe Ile Val Thr Gly Leu Lys
   2065 2070 2075 2080
   Arg Met Ala Val Thr Ile Gly Glu Gln Ala Gln Arg Arg Gly Arg Val
   2085 2090 2095
   Gly Arg Val Lys Pro Gly Arg Tyr Tyr Arg Ser Gln Glu Thr Pro Val
   2100 2105 2110
   Gly Ser Lys Asp Tyr His Tyr Asp Leu Leu Gln Ala Gln Arg Tyr Gly
   2115 2120 2125
   Ile Glu Asp Gly Ile Asn Ile Thr Lys Ser Phe Arg Glu Met Asn Tyr
   2130 2135 2140
   Asp Trp Ser Leu Tyr Glu Glu Asp Ser Leu Met Ile Thr Gln Leu Glu
   2145 2150 2155 2160
   Ile Leu Asn Asn Leu Leu Ile Ser Glu Glu Leu Pro Met Ala Val Lys
   2165 2170 2175
   Asn Ile Met Ala Arg Thr Asp His Pro Glu Pro Ile Gln Leu Ala Tyr
   2180 2185 2190
   Asn Ser Tyr Glu Thr Gln Val Pro Val Leu Phe Pro Lys Ile Arg Asn
   2195 2200 2205
   Gly Glu Val Thr Asp Thr Tyr Asp Asn Tyr Thr Phe Leu Asn Ala Arg
   2210 2215 2220
   Lys Leu Gly Asp Asp Val Pro Pro Tyr Val Tyr Ala Thr Glu Asp Glu
   2225 2230 2235 2240
   Asp Leu Ala Val Glu Leu Leu Gly Leu Asp Trp Pro Asp Pro Gly Asn
   2245 2250 2255
   Gln Gly Thr Val Glu Ala Gly Arg Ala Leu Lys Gln Val Val Gly Leu
   2260 2265 2270
   Ser Thr Ala Glu Asn Ala Leu Leu Val Ala Leu Phe Gly Tyr Val Gly
   2275 2280 2285
   Tyr Gln Ala Leu Ser Lys Arg His Ile Pro Val Val Thr Asp Ile Tyr
   2290 2295 2300
   Ser Val Glu Asp His Arg Leu Glu Asp Thr Thr His Leu Gln Tyr Ala
   2305 2310 2315 2320
   Pro Asn Ala Ile Lys Thr Glu Gly Lys Glu Thr Glu Leu Lys Glu Leu
   2325 2330 2335
   Ala Gln Gly Asp Val Gln Arg Cys Val Glu Ala Val Thr Asn Tyr Ala
   2340 2345 2350
   Arg Glu Gly Ile Gln Phe Met Lys Ser Gln Ala Leu Lys Val Arg Glu
   2355 2360 2365
   Thr Pro Thr Tyr Lys Glu Thr Met Asn Thr Val Ala Asp Tyr Val Lys
   2370 2375 2380
   Lys Phe Ile Glu Ala Leu Thr Asp Ser Lys Glu Asp Ile Ile Lys Tyr
   2385 2390 2395 2400
   Gly Leu Trp Gly Ala His Thr Ala Leu Tyr Lys Ser Ile Gly Ala Arg
   2405 2410 2415
   Leu Gly His Glu Thr Ala Phe Ala Thr Leu Val Val Lys Trp Leu Ala
   2420 2425 2430
   Phe Gly Gly Glu Ser Ile Ser Asp His Ile Lys Gln Ala Ala Thr Asp
   2435 2440 2445
   Leu Val Val Tyr Tyr Ile Ile Asn Arg Pro Gln Phe Pro Gly Asp Thr
   2450 2455 2460
   Glu Thr Gln Gln Glu Gly Arg Lys Phe Val Ala Ser Leu Leu Val Ser
   2465 2470 2475 2480
   Ala Leu Ala Thr Tyr Thr Tyr Lys Ser Trp Asn Tyr Asn Asn Leu Ser
   2485 2490 2495
   Lys Ile Val Glu Pro Ala Leu Ala Thr Leu Pro Tyr Ala Ala Lys Ala
   2500 2505 2510
   Leu Lys Leu Phe Ala Pro Thr Arg Leu Glu Ser Val Val Ile Leu Ser
   2515 2520 2525
   Thr Ala Ile Tyr Lys Thr Tyr Leu Ser Ile Arg Arg Gly Lys Ser Asp
   2530 2535 2540
   Gly Leu Leu Gly Thr Gly Val Ser Ala Ala Met Glu Ile Met Ser Gln
   2545 2550 2555 2560
   Asn Pro Val Ser Val Gly Ile Ala Val Met Leu Gly Val Gly Ala Val
   2565 2570 2575
   Ala Ala His Asn Ala Ile Glu Ala Ser Glu Gln Lys Arg Thr Leu Leu
   2580 2585 2590
   Met Lys Val Phe Val Lys Asn Phe Leu Asp Gln Ala Ala Thr Asp Glu
   2595 2600 2605
   Leu Val Lys Glu Ser Pro Glu Lys Ile Ile Met Ala Leu Phe Glu Ala
   2610 2615 2620
   Val Gln Thr Val Gly Asn Pro Leu Arg Leu Val Tyr His Leu Tyr Gly
   2625 2630 2635 2640
   Val Phe Tyr Lys Gly Trp Glu Ala Lys Glu Leu Ala Gln Arg Thr Ala
   2645 2650 2655
   Gly Arg Asn Leu Phe Thr Leu Ile Met Phe Glu Ala Val Glu Leu Leu
   2660 2665 2670
   Gly Val Asp Ser Glu Gly Lys Ile Arg Gln Leu Ser Ser Asn Tyr Ile
   2675 2680 2685
   Leu Glu Leu Leu Tyr Lys Phe Arg Asp Asn Ile Lys Ser Ser Val Arg
   2690 2695 2700
   Glu Ile Ala Ile Ser Trp Ala Pro Ala Pro Phe Ser Cys Asp Trp Thr
   2705 2710 2715 2720
   Pro Thr Asp Asp Arg Ile Gly Leu Pro His Asp Asn Tyr Leu Arg Val
   2725 2730 2735
   Glu Thr Lys Cys Pro Cys Gly Tyr Arg Met Lys Ala Val Lys Asn Cys
   2740 2745 2750
   Ala Gly Glu Leu Arg Leu Leu Glu Glu Gly Gly Ser Phe Leu Cys Arg
   2755 2760 2765
   Asn Lys Phe Gly Arg Gly Ser Gln Asn Tyr Arg Val Thr Lys Tyr Tyr
   2770 2775 2780
   Asp Asp Asn Leu Ser Glu Ile Lys Pro Val Ile Arg Met Glu Gly His
   2785 2790 2795 2800
   Val Glu Leu Tyr Tyr Lys Gly Ala Thr Ile Lys Leu Asp Phe Asn Asn
   2805 2810 2815
   Ser Lys Thr Val Leu Ala Thr Asp Lys Trp Glu Val Asp His Ser Thr
   2820 2825 2830
   Leu Val Arg Ala Leu Lys Arg Tyr Thr Gly Ala Gly Tyr Arg Gly Ala
   2835 2840 2845
   Tyr Leu Gly Glu Lys Pro Asn His Lys His Leu Ile Gln Arg Asp Cys
   2850 2855 2860
   Ala Thr Ile Thr Lys Asp Lys Val Cys Phe Ile Lys Met Lys Arg Gly
   2865 2870 2875 2880
   Cys Ala Phe Thr Tyr Asp Leu Ser Leu His Asn Leu Thr Arg Leu Ile
   2885 2890 2895
   Glu Leu Val His Lys Asn Asn Leu Glu Asp Arg Glu Ile Pro Ala Val
   2900 2905 2910
   Thr Val Thr Thr Trp Leu Ala Tyr Thr Phe Val Asn Glu Asp Ile Gly
   2915 2920 2925
   Thr Ile Lys Pro Thr Phe Gly Glu Lys Val Thr Pro Glu Lys Gln Glu
   2930 2935 2940
   Glu Val Val Leu Gln Pro Ala Val Val Val Asp Thr Thr Asp Val Ala
   2945 2950 2955 2960
   Val Thr Val Val Gly Glu Thr Ser Thr Met Thr Thr Gly Glu Thr Pro
   2965 2970 2975
   Thr Thr Phe Thr Ser Leu Gly Ser Asp Ser Lys Val Arg Gln Val Leu
   2980 2985 2990
   Lys Leu Gly Val Asp Asp Gly Gln Tyr Pro Gly Pro Asn Gln Gln Arg
   2995 3000 3005
   Ala Ser Leu Leu Glu Ala Ile Gln Gly Val Asp Glu Arg Pro Ser Val
   3010 3015 3020
   Leu Ile Leu Gly Ser Asp Lys Ala Thr Ser Asn Arg Val Lys Thr Ala
   3025 3030 3035 3040
   Lys Asn Val Lys Ile Tyr Arg Ser Arg Asp Pro Leu Glu Leu Arg Glu
   3045 3050 3055
   Met Met Lys Arg Gly Lys Ile Leu Val Val Ala Leu Ser Arg Val Asp
   3060 3065 3070
   Thr Ala Leu Leu Lys Phe Val Asp Tyr Lys Gly Thr Phe Leu Thr Arg
   3075 3080 3085
   Glu Thr Leu Glu Ala Leu Ser Leu Gly Lys Pro Lys Lys Arg Asp Ile
   3090 3095 3100
   Thr Lys Ala Glu Ala Gln Trp Leu Leu Arg Leu Glu Asp Gln Ile Glu
   3105 3110 3115 3120
   Glu Leu Pro Asp Trp Phe Ala Ala Lys Glu Pro Ile Phe Leu Glu Ala
   3125 3130 3135
   Asn Ile Lys Arg Asp Lys Tyr His Leu Val Gly Asp Ile Ala Thr Ile
   3140 3145 3150
   Lys Glu Lys Ala Lys Gln Leu Gly Ala Thr Asp Ser Thr Lys Ile Ser
   3155 3160 3165
   Lys Glu Val Gly Ala Lys Val Tyr Ser Met Lys Leu Ser Asn Trp Val
   3170 3175 3180
   Ile Gln Glu Glu Asn Lys Gln Gly Ser Leu Ala Pro Leu Phe Glu Glu
   3185 3190 3195 3200
   Leu Leu Gln Gln Cys Pro Pro Gly Gly Gln Asn Lys Thr Thr His Met
   3205 3210 3215
   Val Ser Ala Tyr Gln Leu Ala Gln Gly Asn Trp Val Pro Val Ser Cys
   3220 3225 3230
   His Val Phe Met Gly Thr Ile Pro Ala Arg Arg Thr Lys Thr His Pro
   3235 3240 3245
   Tyr Glu Ala Tyr Val Lys Leu Arg Glu Leu Val Asp Glu His Lys Met
   3250 3255 3260
   Lys Ala Leu Cys Gly Gly Ser Gly Leu Ser Lys His Asn Glu Trp Val
   3265 3270 3275 3280
   Ile Gly Lys Val Lys Tyr Gln Gly Asn Leu Arg Thr Lys His Met Leu
   3285 3290 3295
   Asn Pro Gly Lys Val Ala Glu Gln Leu His Arg Glu Gly Tyr Arg His
   3300 3305 3310
   Asn Val Tyr Asn Lys Thr Ile Gly Ser Val Met Thr Ala Thr Gly Ile
   3315 3320 3325
   Arg Leu Glu Lys Leu Pro Val Val Arg Ala Gln Thr Asp Thr Thr Asn
   3330 3335 3340
   Phe His Gln Ala Ile Arg Asp Lys Ile Asp Lys Glu Glu Asn Leu Gln
   3345 3350 3355 3360
   Thr Pro Gly Leu His Lys Lys Leu Met Glu Val Phe Asn Ala Leu Lys
   3365 3370 3375
   Arg Pro Glu Leu Glu Ala Ser Tyr Asp Ala Val Asp Trp Glu Glu Leu
   3380 3385 3390
   Glu Arg Gly Ile Asn Arg Lys Gly Ala Ala Gly Phe Phe Glu Arg Lys
   3395 3400 3405
   Asn Ile Gly Glu Val Leu Asp Ser Glu Lys Asn Lys Val Glu Glu Val
   3410 3415 3420
   Ile Asp Ser Leu Lys Lys Gly Arg Asn Ile Arg Tyr Tyr Glu Thr Ala
   3425 3430 3435 3440
   Ile Pro Lys Asn Glu Lys Arg Asp Val Asn Asp Asp Trp Thr Ala Gly
   3445 3450 3455
   Asp Phe Val Asp Glu Lys Lys Pro Arg Val Ile Gln Tyr Pro Glu Ala
   3460 3465 3470
   Lys Thr Arg Leu Ala Ile Thr Lys Val Met Tyr Lys Trp Val Lys Gln
   3475 3480 3485
   Lys Pro Val Val Ile Pro Gly Tyr Glu Gly Lys Thr Pro Leu Phe Gln
   3490 3495 3500
   Ile Phe Asp Lys Val Lys Lys Glu Trp Asp Gln Phe Gln Asn Pro Val
   3505 3510 3515 3520
   Ala Val Ser Phe Asp Thr Lys Ala Trp Asp Thr Gln Val Thr Thr Arg
   3525 3530 3535
   Asp Leu Glu Leu Ile Arg Asp Ile Gln Lys Phe Tyr Phe Lys Lys Lys
   3540 3545 3550
   Trp His Lys Phe Ile Asp Thr Leu Thr Lys His Met Ser Glu Val Pro
   3555 3560 3565
   Val Ile Ser Ala Asp Gly Glu Val Tyr Ile Arg Lys Gly Gln Arg Gly
   3570 3575 3580
   Ser Gly Gln Pro Asp Thr Ser Ala Gly Asn Ser Met Leu Asn Val Leu
   3585 3590 3595 3600
   Thr Met Val Tyr Ala Phe Cys Glu Ala Thr Gly Val Pro Tyr Lys Ser
   3605 3610 3615
   Phe Asp Arg Val Ala Lys Ile His Val Cys Gly Asp Asp Gly Phe Leu
   3620 3625 3630
   Ile Thr Glu Arg Ala Leu Gly Glu Lys Phe Ala Ser Lys Gly Val Gln
   3635 3640 3645
   Ile Leu Tyr Glu Ala Gly Lys Pro Gln Lys Ile Thr Glu Gly Asp Lys
   3650 3655 3660
   Met Lys Val Ala Tyr Gln Phe Asp Asp Ile Glu Phe Cys Ser His Thr
   3665 3670 3675 3680
   Pro Val Gln Val Arg Trp Ser Asp Asn Thr Ser Ser Tyr Met Pro Gly
   3685 3690 3695
   Arg Asn Thr Thr Thr Ile Leu Ala Lys Met Ala Thr Arg Leu Asp Ser
   3700 3705 3710
   Ser Gly Glu Arg Gly Thr Ile Ala Tyr Glu Lys Ala Val Ala Phe Ser
   3715 3720 3725
   Phe Leu Leu Met Tyr Ser Trp Asn Pro Leu Ile Arg Arg Ile Cys Leu
   3730 3735 3740
   Leu Val Leu Ser Thr Glu Leu Gln Val Arg Pro Gly Lys Ser Thr Thr
   3745 3750 3755 3760
   Tyr Tyr Tyr Glu Gly Asp Pro Ile Ser Ala Tyr Lys Glu Val Ile Gly
   3765 3770 3775
   His Asn Leu Phe Asp Leu Lys Arg Thr Ser Phe Glu Lys Leu Ala Lys
   3780 3785 3790
   Leu Asn Leu Ser Met Ser Thr Leu Gly Val Trp Thr Arg His Thr Ser
   3795 3800 3805
   Lys Arg Leu Leu Gln Asp Cys Val Asn Val Gly Thr Lys Glu Gly Asn
   3810 3815 3820
   Trp Leu Val Asn Ala Asp Arg Leu Val Ser Ser Lys Thr Gly Asn Arg
   3825 3830 3835 3840
   Tyr Ile Pro Gly Glu Gly His Thr Leu Gln Gly Lys His Tyr Glu Glu
   3845 3850 3855
   Leu Ile Leu Ala Arg Lys Pro Ile Gly Asn Phe Glu Gly Thr Asp Arg
   3860 3865 3870
   Tyr Asn Leu Gly Pro Ile Val Asn Val Val Leu Arg Arg Leu Lys Ile
   3875 3880 3885
   Met Met Met Ala Leu Ile Gly Arg Gly Val
   3890 3895
(2) INFORMATION FOR SEQ ID NO:3:
   (i) SEQUENCE CHARACTERISTICS:
      (A) LENGTH: 33 base pairs
      (B) TYPE: nucleic acid
      (C) STRANDEDNESS: single
      (D) TOPOLOGY: linear
   (ix) FEATURE:
      (A) NAME/KEY: -
      (B) LOCATION: 1..33
      (D) OTHER INFORMATION: /label= primer_1
   (xi) SEQUENCE DESCRIPTION: SEQ ID NO:3:
   CCTACTAACC ACGTTAAGTG CTGTGACTTT AAA 33
(2) INFORMATION FOR SEQ ID NO:4:
   (i) SEQUENCE CHARACTERISTICS:
      (A) LENGTH: 39 base pairs
      (B) TYPE: nucleic acid
      (C) STRANDEDNESS: single
      (D) TOPOLOGY: linear
   (ix) FEATURE:
      (A) NAME/KEY: -
      (B) LOCATION: 1..39
      (D) OTHER INFORMATION: /label= primer_2
   (xi) SEQUENCE DESCRIPTION: SEQ ID NO:4:
   TTCTGTTCTC AAGGTTGTGG GGCTCACTGC TGTGCACTC 39
(2) INFORMATION FOR SEQ ID NO:5:
   (i) SEQUENCE CHARACTERISTICS:
      (A) LENGTH: 16 base pairs
      (B) TYPE: nucleic acid
      (C) STRANDEDNESS: single
      (D) TOPOLOGY: linear
   (ix) FEATURE:
      (A) NAME/KEY: -
      (B) LOCATION: 1..16
      (D) OTHER INFORMATION: /label= Adaptor_1 /note= "Upper strand of Bam HI - Hinf I adaptor, containing ATG at 364-366"
   (xi) SEQUENCE DESCRIPTION: SEQ ID NO:5:
   GATCCACCAT GGAGTT 16
(2) INFORMATION FOR SEQ ID NO:6:
   (i) SEQUENCE CHARACTERISTICS:
      (A) LENGTH: 16 base pairs
      (B) TYPE: nucleic acid
      (C) STRANDEDNESS: single
      (D) TOPOLOGY: linear
   (ix) FEATURE:
      (A) NAME/KEY: -
      (B) LOCATION: 1..16
      (D) OTHER INFORMATION: /label= Adaptor_2 /note= "Lower strand of Bam HI - Hinf I adaptor, containing ATG at 364-366"
   (xi) SEQUENCE DESCRIPTION: SEQ ID NO:6:
   GTGGTACCTC AACTTA 16
(2) INFORMATION FOR SEQ ID NO:7:
   (i) SEQUENCE CHARACTERISTICS:
      (A) LENGTH: 10 base pairs
      (B) TYPE: nucleic acid
      (C) STRANDEDNESS: double
      (D) TOPOLOGY: linear
   (ix) FEATURE:
      (A) NAME/KEY: -
      (B) LOCATION: 1..10
      (D) OTHER INFORMATION: /label= Adaptor_3 /note= "Double stranded Stu I - Eco RI blunt adaptor"
   (xi) SEQUENCE DESCRIPTION: SEQ ID NO:7:
   GCCTGAATTC 10
(2) INFORMATION FOR SEQ ID NO: 8:
   (i) SEQUENCE CHARACTERISTICS:
      (A) LENGTH: 300 base pairs
      (B) TYPE: nucleic acid
      (C) STRANDEDNESS: double
      (D) TOPOLOGY: linear
   (ii) MOLECULE TYPE: cDNA
   (vii) IMMEDIATE SOURCE:
      (B) CLONE: lambda gt11 clone
   (ix) FEATURE:
      (A) NAME/KEY: CDS
      (B) LOCATION: 1..300
      (D) OTHER INFORMATION: /note= "Part of 0.8 kb insert of Lambda gt11"
   (xi) SEQUENCE DESCRIPTION: SEQ ID NO:12:
(2) INFORMATION FOR SEQ ID NO: 9:
   (i) SEQUENCE CHARACTERISTICS:
      (A) LENGTH: 100 amino acids
      (B) TYPE: amino acid
      (D) TOPOLOGY: linear
   (ii) MOLECULE TYPE: protein
   (xi) SEQUENCE DESCRIPTION: SEQ ID NO:13:

## Description

The present invention is concerned with a nucleic acid sequence, a recombinant nucleic acid molecule comprising such a nucleic acid sequence, a recombinant expression system comprising such a recombinant nucleic acid molecule, a polypeptide characteristic of the hog cholera virus, a vaccine comprising such a polypeptide or recombinant expression system as well as a method for the preparation of such vaccines.

Classical swine fever or hog cholera (HC) represents an economically important disease of swine in many countries worldwide. Under natural conditions, the pig is the only animal known to be susceptible to HC. Hog cholera is a highly contagious disease which causes degeneration in the walls of capillaries, resulting in hemorrhages and necrosis of the internal organs. In the first instance hog cholera is characterized by fever, anorexia, vomiting and diarrhea which can be followed by a chronic course of the disease characterized by infertility, abortion and weak offsprings of sows. However, nearly all pigs die within 2 weeks after the first symptoms appear.

The causative agent, the hog cholera virus (HCV) has been shown to be structurally and serologically related to bovine viral diarrhea virus (BVDV) of cattle and to border disease virus (BDV) of sheep. These viruses are grouped together into the genus pestivirus within the family togaviridae. The nature of the genetic material of pestiviruses has long been known to be RNA, i.e. positive-strand RNA which lacks significant polyadenylation. The HCV probably comprises 3-5 structural proteins of which two are possibly glycosylated. The number of non-structural viral proteins is unknown.

Modified HCV vaccines (comprising attenuated or killed viruses) for combating HC infection have been developed and are presently used. However, infection of tissue culture cells to obtain HCV material to be used in said modified virus vaccines, leads to low virus yields and the virions are hard to purify. Modified live virus vaccines always involve the risk of inoculating animals with partially attenuated pathogenic HCV which is still pathogenic and can cause disease in the inoculated animal or offspring and of contamination by other viruses in the vaccine. In addition the attenuated virus may revert to a virulent state.

There are also several disadvantages using inactivated vaccines, e.g. the risk of only partial inactivation of viruses, the problem that only a low level of immunity is achieved requiring additional immunizations and the problem that antigenic determinants are altered by the inactivation treatment leaving the inactivated virus less immunogenic.

Furthermore, the usage of modified HCV vaccines is not suited for eradication programmes.

Until now, according to our knowledge diagnostic tests in swine which can distinguish between HCV or BVDV infection are not available. This is important as BVDV infection in pigs is of lower significance than HCV infection which means that BVDV infected pigs do not have to be eradicated.

Vaccines containing only the necessary and relevant HCV immunogenic material which is capable of eliciting an immune response against the pathogen do not display abovementioned disadvantages of modified vaccines.

According to the present invention a nucleic acid sequence encoding a polypeptide characteristic of hog cholera virus has been found. Fragments of said nucleic acid sequence or said polypeptide are also within the present invention. Both the nucleic acid sequence and the polypeptide or fragments thereof can be used for the preparation of a vaccine containing only the necessary and relevant immunogenic material for immunizing animals against HCV infection. "Nucleic acid sequence" refers both to a ribonucleic acid sequence and a deoxy-ribonucleic acid sequence.

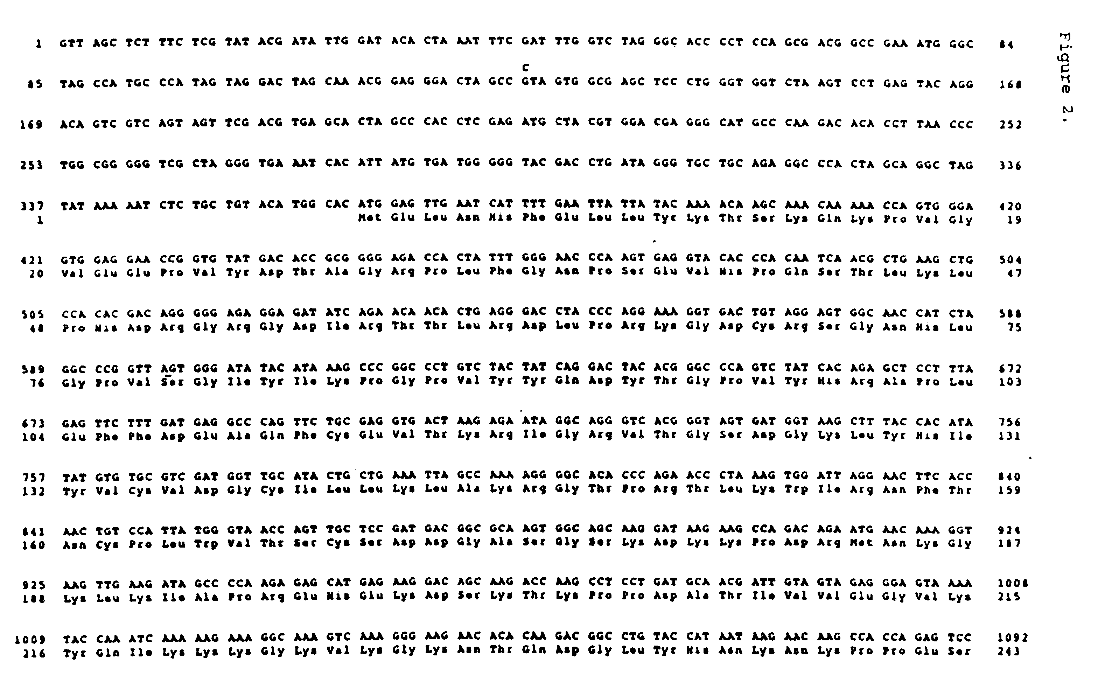

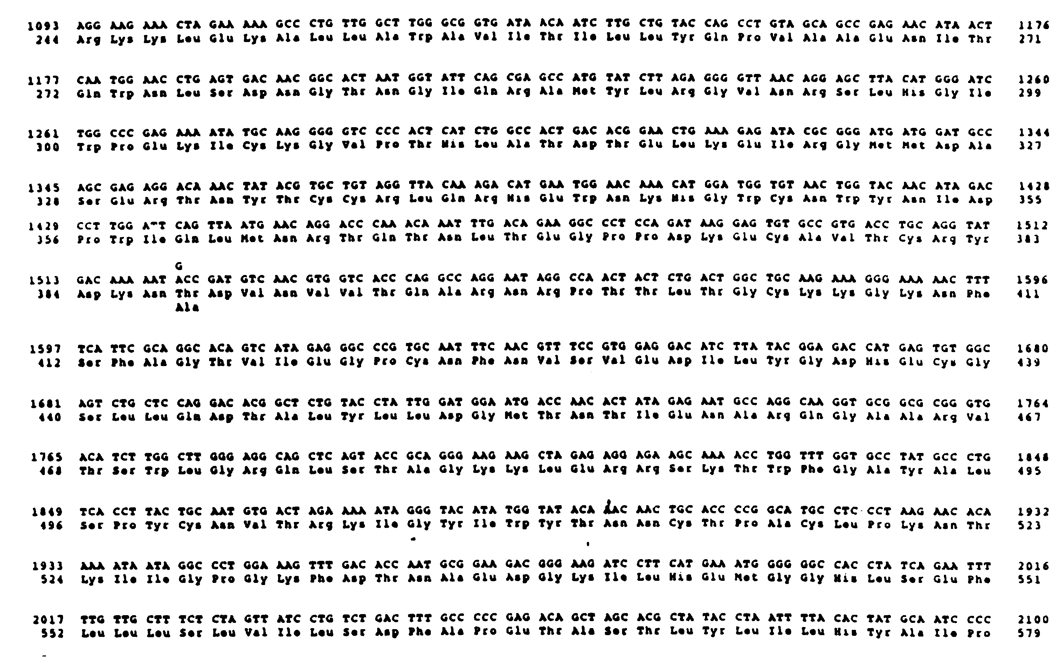

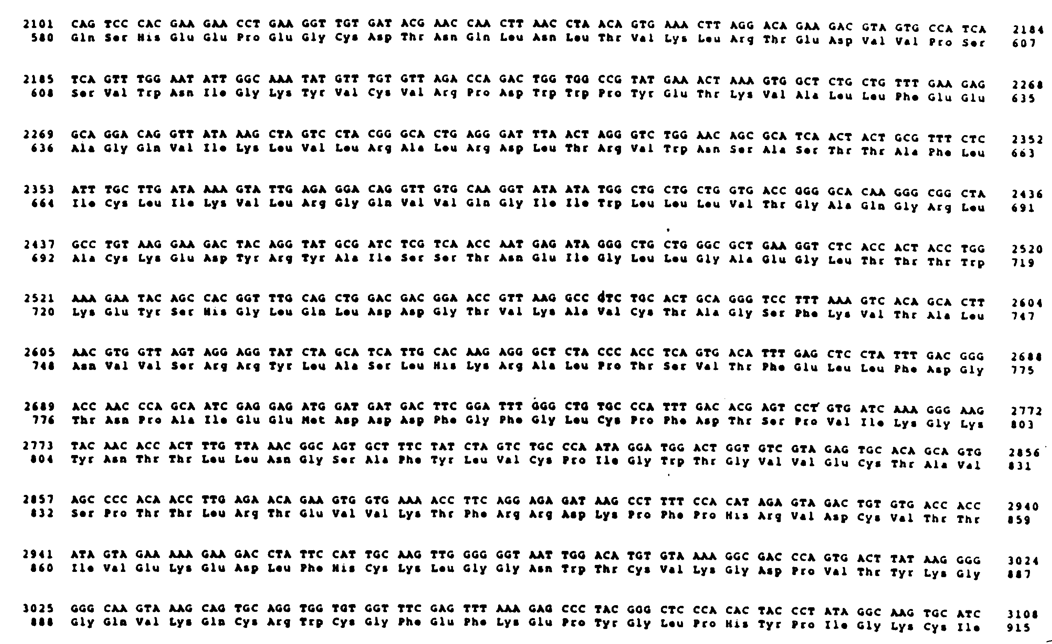

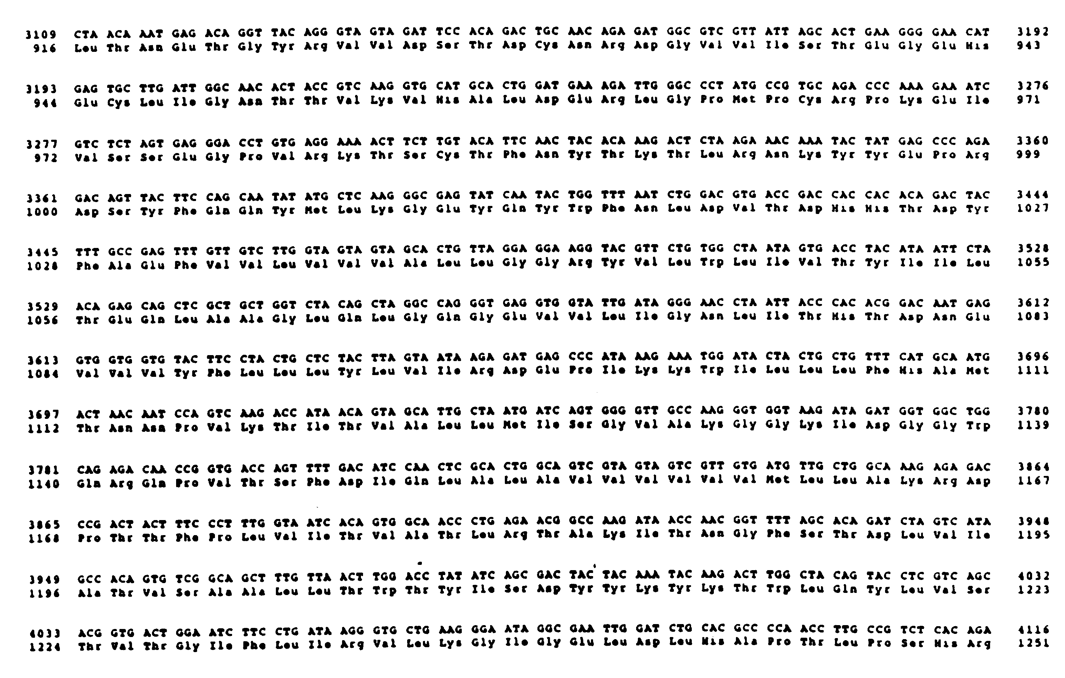

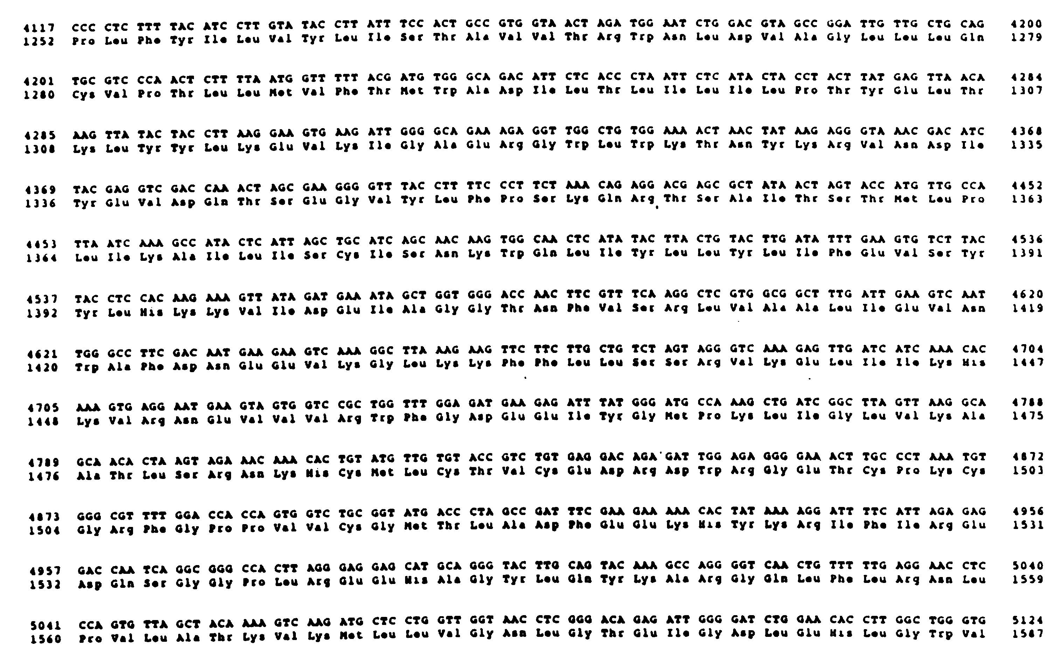

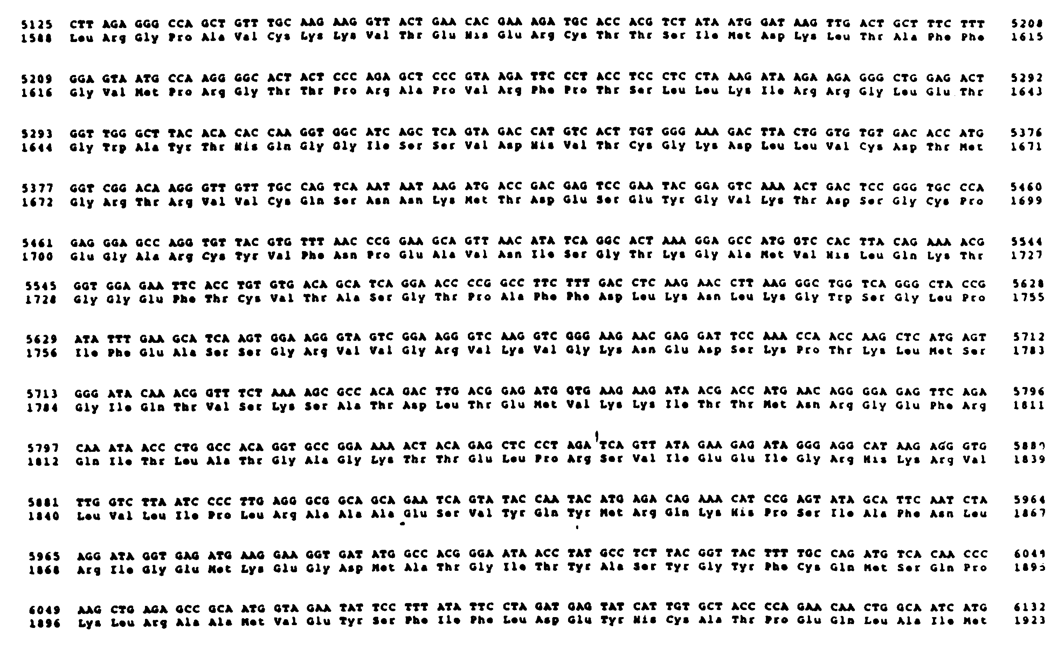

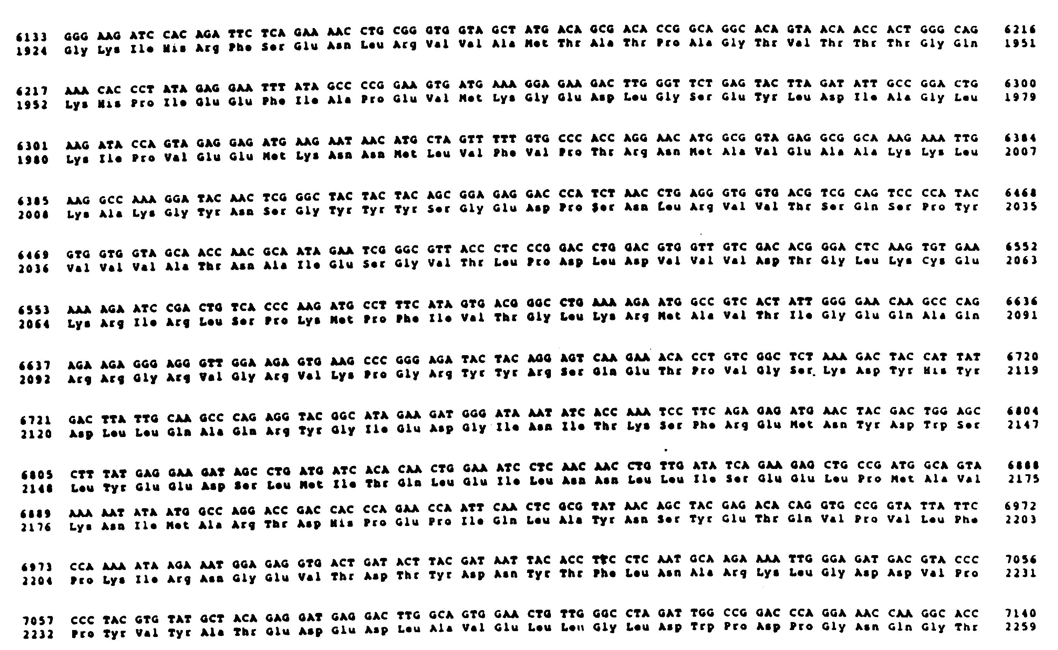

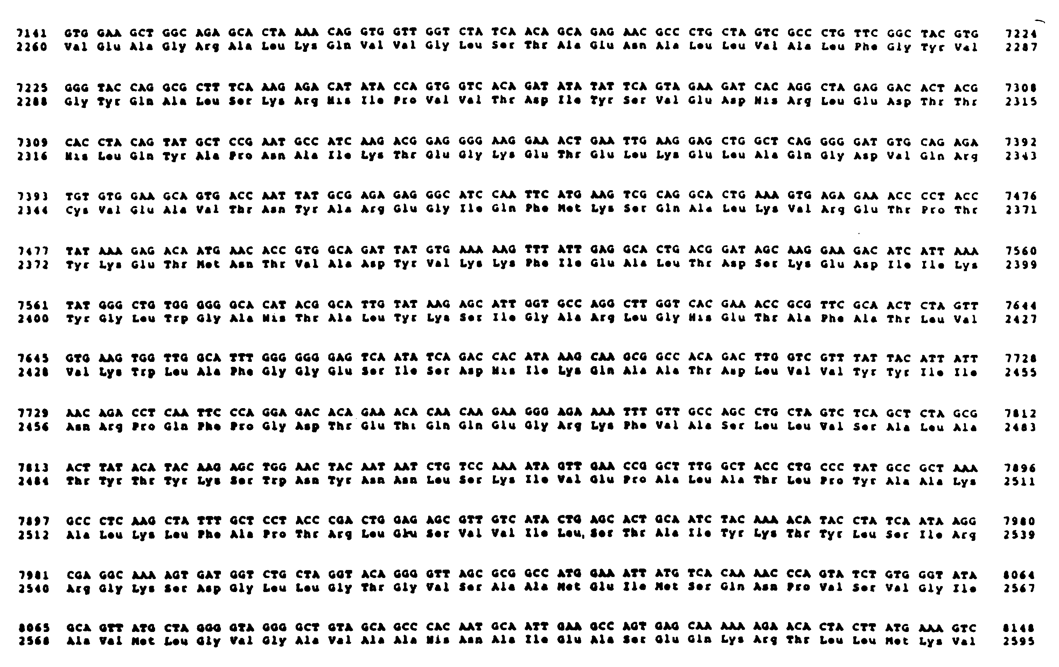

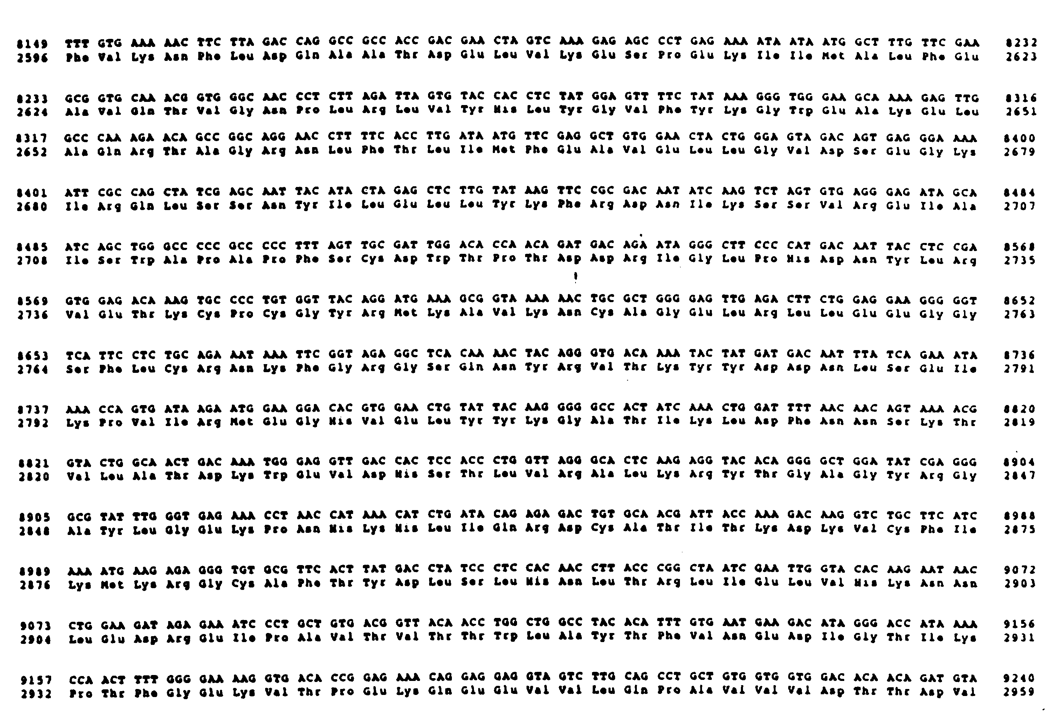

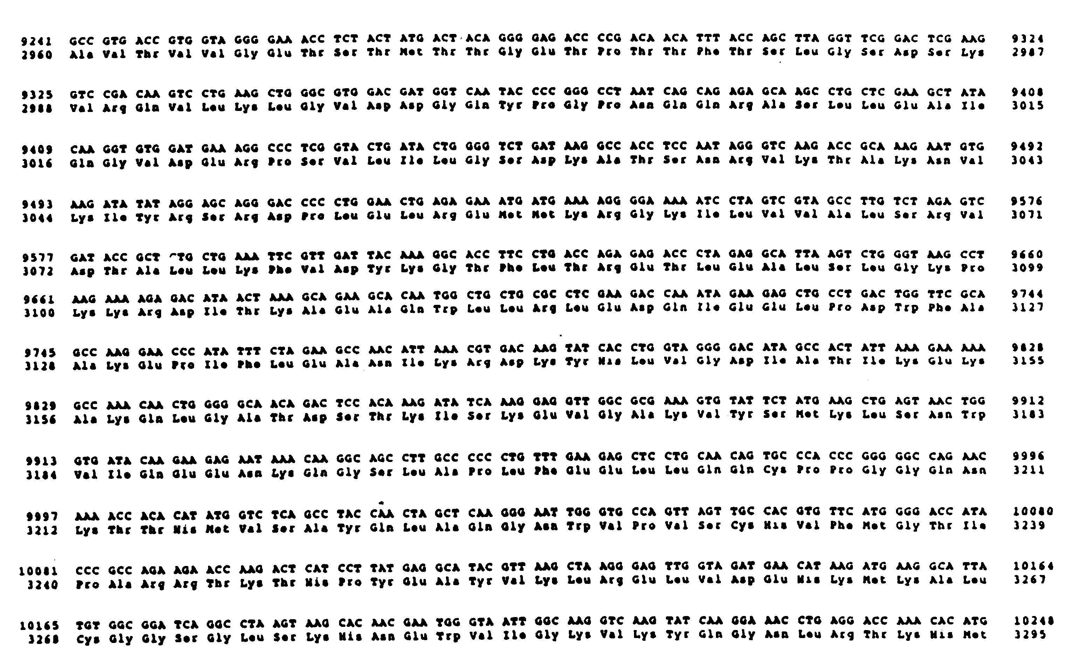

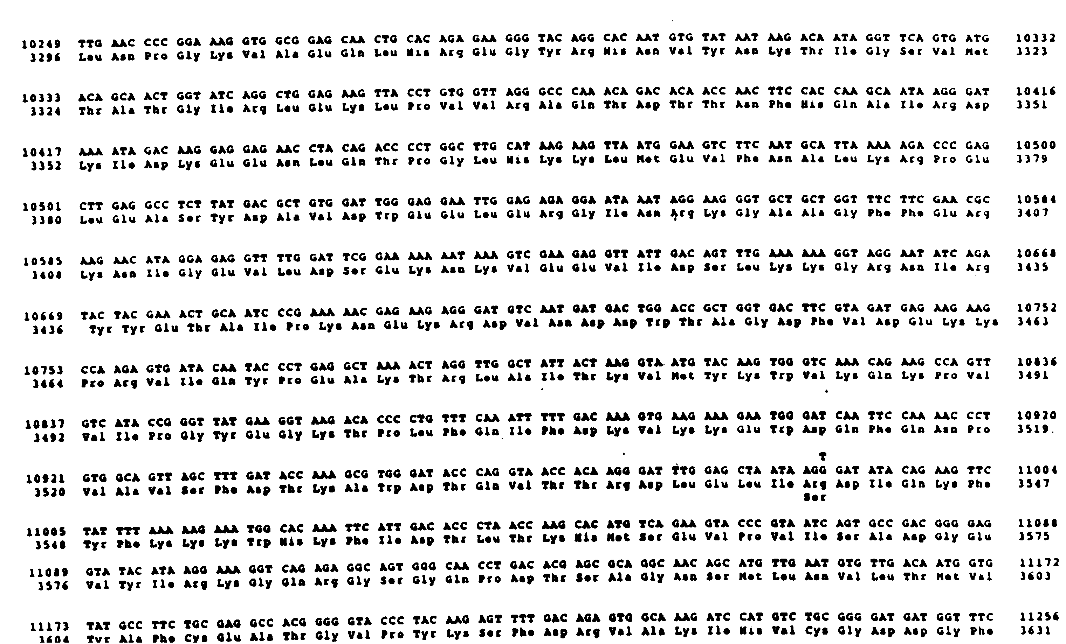

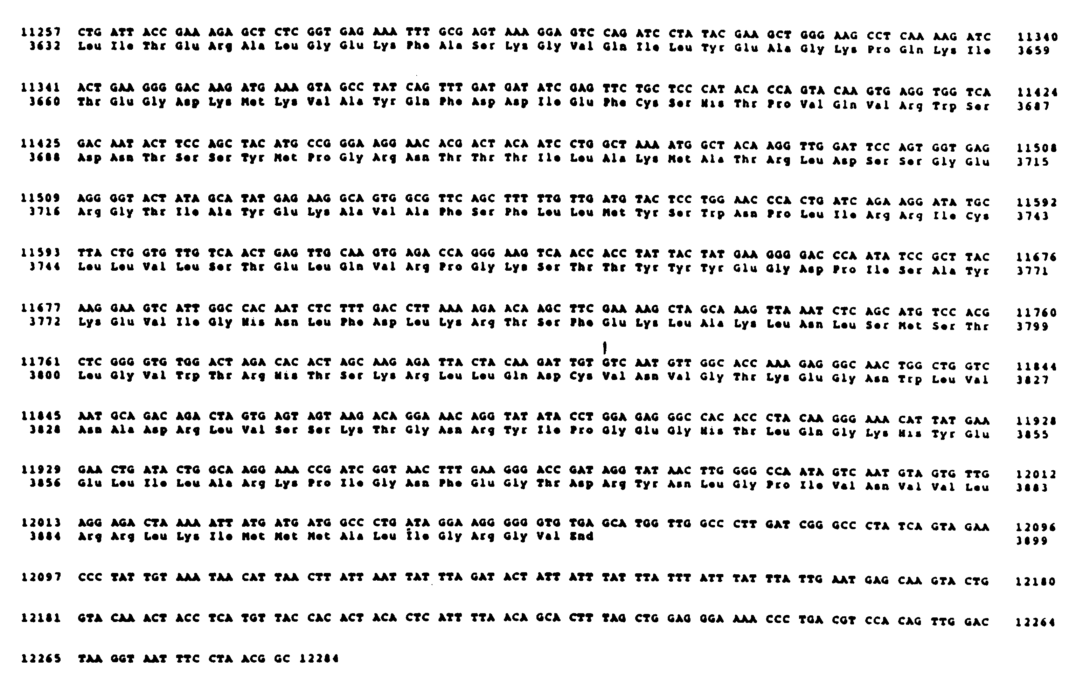

A nucleic acid sequence according to the present invention is shown in figure 2 (SEQ ID NO: 1). As is well known in the art, the degeneracy of the genetic code permits substitution of bases in a codon resulting in an other codon but still coding for the same amino acid, e.g. the codon for the amino acid glutamic acid is both GAT and GAA. Consequently, it is clear that for the expression of a polypeptide with the amino acid sequence shown in figure 2 (SEQ ID NO: 1-2) use can be made of a nucleic acid sequence with such an alternative codon composition different from the nucleic acid sequence shown in figure 2 (SEQ ID NO: 1).

Also included within the scope of the invention are nucleic acid sequences which hybridize under stringent conditions to the nucleic acid sequence shown in figure 2 (SEQ ID NO: 1). These nucleic acid sequences are related to the nucleic acid sequence shown in figure 2 (SEQ ID NO: 1) but may comprise nucleotide substitutions, mutations, insertions, deletions etc. and encode polypeptides which are functionally equivalent to the polypeptide shown in figure 2 (SEQ ID NO: 1-2), i.e. the amino acid sequence of a related polypeptide is not identical with the amino acid sequence shown in figure 2 (SEQ ID NO: 1-2) but features corresponding immunological properties characteristic for HCV.

Within the scope of the invention are also polypeptides encoded by such related nucleic acid sequences.

The nucleic acid sequence shown in figure 2 (SEQ ID NO: 1) is a cDNA sequence derived from the genomic RNA of HCV. This continuous sequence is 12284 nucleotides in length, and contains one long open reading frame (ORF), starting with the ATG codon at position 364 to 366 and ending with a TGA codon as a translational stop codon at position 12058 to 12060. This ORF consists of 3898 codons capable of encoding 435 kDa of protein.

In vivo, during HCV replication in an infected cell, this protein is synthesized as a polyprotein precursor molecule which is subsequently processed to fragment polypeptides by (enzymatic) cleavage of the precursor molecule. These fragments form after possible post-translational modifications the structural and non-structural proteins of the virus. A preferred nucleic acid sequence contains the genetic information for such a fragment with immunizing properties against HCV or immunological properties characteristic for HCV or contains the genetic information for a portion of such a fragment which still has the immunizing properties or the immunological properties characteristic for HCV.

Fragment polypeptides of the polypeptide according to figure 2 (SEQ ID NO: 1-2) and the portions thereof, which can be used for the immunisation of animals against HC or for diagnosis of HC also form part of the present invention.
A fragment-coding region is located within the amino acid position about 1-249, 263-487, 488-688 or 689-1067.
The 1-249 region essentially represents the core protein whereas the 263-487, 488-688 and 689-1067 regions essentially represent glycoproteins of 33 kD, 44/48 kD and 55 kD respectively. Within the scope of the invention are also nucleic acid sequences comprising the genetic information for one or more of the coding regions mentioned above or portions thereof.

A preferred region to be incorporated into a vaccine against HCV infection is the region corresponding to the 55 kD protein of HCV or a portion thereof still having immunizing activity.

Furthermore, a nucleic acid sequence at least comprising the coding sequences for said 55 kD protein or portion thereof can advantageously be applied according to the present invention.

In addition, a preferred portion of the HCV 55 kD protein, which can be used for immunization of pigs against HCV infection, is determined by analyses of HCV deletion mutants with anti-55 kD protein monoclonal antibodies having virus neutralizing activity. Such a portion comprising an epitope spans the amino acid sequence about 812-859 and is coded by the nucleotide sequence about 2799-2938. A polypeptide at least comprising said amino acid sequence or a nucleic acid sequence at least comprising said nucleotide sequence form part of the present invention too.

A nucleic acid sequence according to the invention which can be used for the diagnosis of HCV infection in pigs and which can be applied to discriminate HCV from BVDV can be derived from the gene encoding the 55 kD protein.

Preferably, such a nucleic acid sequence is derived from the nucleotide sequences 2587-2619 or 2842-2880, both sequences being part of the gene encoding the 55 kD protein. A preferred oligonucleotide for diagnostic purposes is (SEQ ID NO: 3 and 4, respectively):

Moreover, a nucleic acid sequence comprising at least a sub-sequence of said oligonucleotides and which still can be used to differentiate between HCV and BVDV forms part of the invention.

The invention also relates to a test kit to be used in an assay, this test kit containing a nucleic acid sequence according to the invention.

Preferably the test kit comprises an oligonucleotide mentioned above or a nucleic acid sequence comprising at least a sub-sequence thereof.

Variations or modifications in the polypeptide shown in figure 2 (SEQ ID NO: 1-2) or fragments thereof, such as natural variations between different strains or other derivatives, are possible while retaining the same immunologic properties. These variations may be demonstrated by (an) amino acid difference(s) in the overall sequence or by deletions, substitutions, insertions, inversions or additions of (an) amino acid(s) in said polypeptide.

Moreover, the potential exists, in the use of recombinant DNA technology, for the preparation of various derivatives of the polypeptide shown in figure 2 (SEQ ID NO: 1-2) or fragments thereof, variously modified by resultant single or multiple amino acid substitutions, deletions, additions or replacements, for example by means of site directed mutagenesis of the underlying DNA. All such modifications resulting in derivatives of the polypeptide shown in figure 2 (SEQ ID NO: 1-2) or fragments thereof are included within the scope of the present invention so long as the essential characteristic activity of said polypeptide or fragment thereof, remains unaffected in essence.

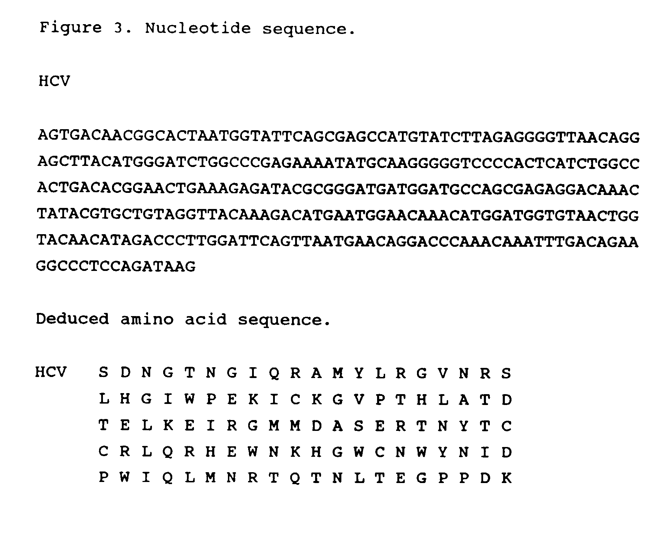

RNA isolated from pelleted virions was isolated and used for the synthesis of cDNA. This cDNA was cloned in phage λgt11 and the respective library was amplified and screened with goat anti-HCV antiserum. Two positive clones could be identified and shown to have inserts with sizes of 0,8 kb and 1,8 kb. The 0,8 kb λgt11 insert was partially sequenced (see figure 3, SEQ ID NO: 8-9) and determined to be located between about 1,2 and 2,0 kb on the HCV genome (see figure 2).

A nucleic acid sequence according to the invention which can be used for the diagnosis of HCV in infected animals and which surprisingly can be applied to discriminate HCV from BVDV is represented by the nucleotide sequence 5551-5793 shown in figure 2 (SEQ ID NO: 1).

Moreover, a nucleic acid sequence comprising at least a sub-sequence of said nucleotide sequence and which still can be used to differentiate between HCV and BVDV forms part of the invention.

The invention also relates to a test kit to be used in an assay, this test kit containing a nucleic acid sequence according to the invention.

Preferably the test kit comprises the nucleic acid sequence represented by the nucleotide sequence 5551-5793 shown in figure 2 (SEQ ID NO: 1) or a nucleic acid sequence comprising at least a sub-sequence thereof mentioned above.

RNA isolated from pelleted virions was isolated and used for the synthesis of cDNA. This cDNA was cloned in phage λgt11 and the respective library was amplified and screened with goat anti-HCV antiserum. Two positive clones could be identified and shown to have inserts with sizes of 0,8 kb and 1,8 kb. The 0,8 kb λgt11 insert was partially sequenced (see figure 3, SEQ ID NO: 8-9) and determined to be located between about 1,2 and 2,0 kb on the HCV genome (see figure 2).

A nucleic acid sequence according to the present invention can be ligated to various vector nucleic acid molecules such as plasmid DNA, bacteriophage DNA or viral DNA to form a recombinant nucleic acid molecule. The vector nucleic acid molecules preferably contain DNA sequences to initiate, control and terminate transcription and translation. A recombinant expression system comprising a host containing such a recombinant nucleic acid molecule can be used to allow for a nucleic acid sequence according to the present invention to express a polypeptide encoded by said nucleic acid sequence. The host of above-mentioned recombinant expression system can be of procaryotic origin, e.g. bacteria such as E.coli, B.subtilis and Pseudomonas, viruses such as vaccinia and fowl pox virus or eucaryotic origin such as yeasts or higher eucaryotic cells such as insect, plant or animal cells.

Immunization of animals against HC can, for example, be achieved by administering to the animal a polypeptide according to the invention as a so-called "sub-unit" vaccine. The subunit vaccine according to the invention comprises a polypeptide generally in a pure form, optionally in the presence of a pharmaceutically acceptable carrier.

Small fragments are preferably conjugated to carrier molecules in order to raise their immunogenicity. Suitable carriers for this purpose are macromolecules, such as natural polymers (proteins, like key hole limpet hemocyanin, albumin, toxins), synthetic polymers like polyamino acids (polylysine, polyalanine), or micelles of amphiphilic compounds like saponins. Alternatively these fragments may be provided as polymers thereof, preferably linear polymers. Polypeptides to be used in such subunit vaccines can be prepared by methods known in the art, e.g. by isolation said polypeptides from hog cholera virus, by recombinant DNA techniques or by chemical synthesis.

If required the polypeptides according to the invention to be used in a vaccine can be modified in vitro or in vivo, for example by glycosylation, amidation, carboxylation or phosphorylation.

An alternative to subunit vaccines are "vector" vaccines. A nucleic acid sequence according to the invention is integrated by recombinant techniques into the genetic material of another micro-organism (e.g. virus or bacterium) thereby enabling the micro-organism to express a polypeptide according to the invention. This recombinant expression system is administered to the animal to be immunized whereafter it replicates in the inoculated animal and expresses the polypeptide resulting in the stimulation of the immune system of the animal. Suitable examples of vaccine vectors are pox viruses (such as vaccinia, cow pox, rabbit pox), avian pox viruses (such as fowl pox virus) pseudorabies virus, adeno viruses, influenza viruses, bacteriophages or bacteria (such as Escherichia coli and Salmonella).

The recombinant expression system having a nucleic acid sequence according to the invention inserted in its nucleic acid sequence can for example be grown in a cell culture and can if desired be harvested from the infected cells and formed to a vaccine optionally in a lyophilized form. Said genetically manipulated micro-organism can also be harvested from live animals infected with said micro-organism. Abovementioned recombinant expression system can also be propagated in a cell culture expressing a polypeptide according to the invention, whereafter the polypeptide is isolated from the culture.

A vaccine comprising a polypeptide or a recombinant expression system according to the present invention can be prepared by procedures well-known in the art for such vaccines. A vaccine according to the invention can consist inter alia of whole host, host extract, partially or completely purified polypeptide or a partially or completely purified recombinant expression system as above-mentioned.

The vaccine according to the invention can be administered in a conventional active immunization scheme: single or repeated administration in a manner compatible with the dosage formulation and in such amount as will be therapeutically effective and immunogenic. The administration of the vaccine can be done, e.g. intradermally, subcutaneously, intramusculary, intra-venously or intranasally. For parenteral administration the vaccines may additionally contain a suitable carrier, e.g. water, saline or buffer solution with or without adjuvants, stabilizers, solubilizers, emulsifiers etc.

The vaccine may additionally contain immunogens related to other diseases or nucleic acid sequences encoding these immunogens like antigens of parvovirus, pseudorabies virus, swine influenza virus, TGE virus, rotavirus, Escherichia coli, Bordetella, Pasteurella, Erysipelas etc. to produce a multivalent vaccine.

Polypeptides according to the present invention can also be used in diagnostic methods to detect the presence of HCV antigen or antibody in an animal. Moreover, nucleic acid sequences according to the invention can be used to produce polypeptides to be used in above-mentioned diagnostic methods or as a hybridisation probe for the detection of the presence of HCV nucleic acid in a sample.

### Example 1

### Immunological identification of cDNA clones

Infection of cells and harvesting of virus. PK15 and 38A₁D cells were grown in DMEM with 10% FCS and were infected in suspension by the virulent HCV strain Alfort in a volume of 20-30 ml at a cell concentration of 5 x 10⁷/ml at 37 ^{o}C for 90 min with an m.o.i. of 0.01 to 0.001 (as determined by immunofluorescence assay). Thereafter, the PK15 cells were seeded in tissue culture plates (150 mm diameter), while the suspension cells 38A₁D were incubated in bottles with gentle stirring (Tecnomara, Switzerland). For cDNA synthese, the tissue culture supernatant was harvested 48 hours after infection, clarified at 12,000 g, and afterwards the virus pelleted in a TFA 20 rotor (Contron, Italy) at 54,000 g for 12 hours.

Preparation of goat anti-HCV serum. A fibroblastic cell strain was established from the skin biopsy of a young goat by standard cell culture techniques. The cells were initially grown in F-10 medium with 10% FCS and later in DMEM with 10% FCS. Goat fibroblasts were infected with HCV. Over the first 26 hours p.i., the cells were washed every 8 hours 3 times with PBS and afterwards incubated in DMEM with 10% preimmune goat serum (PGS). 48 hours p.i., the tissue culture supernatant was harvested and used as stock virus. Before immunization, goat cells for 30 tissue culture dishes (150 mm diameter) were kept for 3 passages in medium with 10% PGS and then infected with the stock virus. 48 hours p.i., the goat was immunized with X-ray-inactivated pelleted virus and infected cells. Both were emulsified in Freund's adjuvant (complete for basis immunization, incomplete for booster injections) and injected subcutaneously. To obtain antibodies recognizing denatured molecules, the antigen preparations were incubated in 0.2% SDS, 3 mM DTT at 95 ^{o}C for 5 min before injection.

RNA preparation, cDNA synthesis and cloning. RNA from virions was isolated by using the guanidine thiocyanate method described by Chirgwin et al. (1979). RNA from pelleted virions (5 µg total RNA, approximately 0.5 µg HCV RNA) and 0.1 µg of random hexanucleotide primer (Pharmacia, Sweden) in 20 µl of water were heated to 65 ^{o}C for 10 min, chilled on ice, and adjusted to first strand buffer (50 mM Tris-HCl pH 8.3; 30 mM KCl; 8 mM MgCl₂; 1 mM DTT, dATP, dCTP, dGTP, dTTP 1 mM each and 500 units RNAguard [Pharmacia, Sweden] per ml) in a final volume of 32 µl. 35 units of AMV reverse transcriptase (Life Sciences Inc., USA) were added. After 1 hour at 43 ^{o}C the reaction mixture was added to one vial of second strand synthesis mixture (cDNA synthesis kit, Pharmacia, Sweden). Second strand synthesis, preparation of blunt ends, and Eco RI adaptor ligation and phosphorylation were done as recommended by the supplier.

The cDNA was size-fractionated by preparative agarose gel electrophoresis. The part of the gel containing DNA molecules smaller than 0.5 kb was discarded. The remaining DNA was concentrated by running the gel reversely for 15 min and extracted from the agarose after 3 cycles of freezing and thawing with phenol.

Ethanol co-precipitated cDNA and λgt11 DNA (1 µg EcoRI digested dephosphorylated arms, Promega, USA) was ligated by 3 units of T4 DNA ligase (Pharmacia, Sweden) in a total volume of 10 µl ligase buffer (30 mM Tris-HCl pH 7.4; 10 mM MgCl₂; 10 mM DTT; 1 mM ATP). In vitro packaging with a commercially available extract (Packagene, Promega, USA) and infection of E.coli K12 cells, strain Y 1090, with resulting phages was performed as recommended by the supplier. The library was amplified once as described (Davis et al., 1986).

Screening of λgt11 library. Screening was basically performed as described (Young and Davis, 1983) using the Protoblot system purchased from Promega, USA (Huynh et al., 1985) and a serum dilution of 10⁻³. For background reduction the goat anti HCV serum was treated with E.coli lysate (strain Y1090) at 0.8 mg/ml (Huynh et al., 1985). Two positive clones having inserts of 0.8 kb and 1.8 kb, respectively could be identified.

Nick translation and Northern hybridization. 50 ng of the 0.8 kb HCV nucleic acid sequence labeled with [α³²P]dCTP (3000 Ci per mMole, Amersham Buchler, FRG) by nick translation (nick translation kit, Amersham Buchler, FRG) was hybridized to Northern filters at a concentration of 5 ng per ml of hybridization mixture (5 x SSC; 1 x Denhardt's; 20 mM sodium phosphate pH 6.8; 0.1% SDS and 100 µg yeast tRNA [Boehringer-Mannheim, FRG] per ml) at 68 ^{o}C for 12 to 14 hours. Membranes were then washed as described (Keil et al., 1984) and exposed at -70 ^{o}C to Kodak X-Omat AR films for varying times using Agfa Curix MR 800 intensifying screens.

The 0.8 kb nucleic acid sequence hybridized not only to intact HCV RNA but also to degradation products thereof. The 0.8 kb nucleic acid sequence did not hybridize to the 1.8 kb nucleic acid sequence, indicating that these two nucleic acid sequences correspond with fragments of the HCV genome which are not located in the same region of the genomic RNA.

Nucleotide sequencing. Subcloning of HCV specific phage DNA inserts into plasmid pEMBL 18 plus was done according to standard procedures (Maniatis et al., 1982). Single-stranded DNA of recombinant pEMBL plasmids was prepared as described (Dente et al., 1985). Dideoxy sequencing reactions (Sanger et al., 1977) were carried out as recommended by the supplier (Pharmacia, Sweden).

### Example 2

### Molecular cloning and nucleotide sequence of the genome of HCV

RNA preparation, cDNA synthesis and cloning. RNA preparation, cDNA synthesis, size selection and ligation of co-precipitated cDNA and λgt10 DNA (1 µg EcoRI digested dephosphorylated arms, Promega, USA) were done as described above. In vitro packaging of phage DNA using Packagene (Promega, USA) and titration of phages on E.coli strain C 600 HFL were performed as suggested by the supplier. The library was amplified once (Davis et al., 1986), and replicas transferred to nictrocellulose membranes (Amersham Buchler, FRG) (Benton and Davis, 1977) were hybridized with oligonucleotides as described above for Northern hybridization. Screening with cDNA fragments labeled with [α³²P] dCTP by nick translation (nick translation kit, Amersham Buchler, FRG) was done as described by Benton and Davis (1977). Positive clones were plaque purified and inserts subcloned into pEMBL plasmids (Maniatis et al., 1982; Dente et al., 1985; Davis et al., 1986).

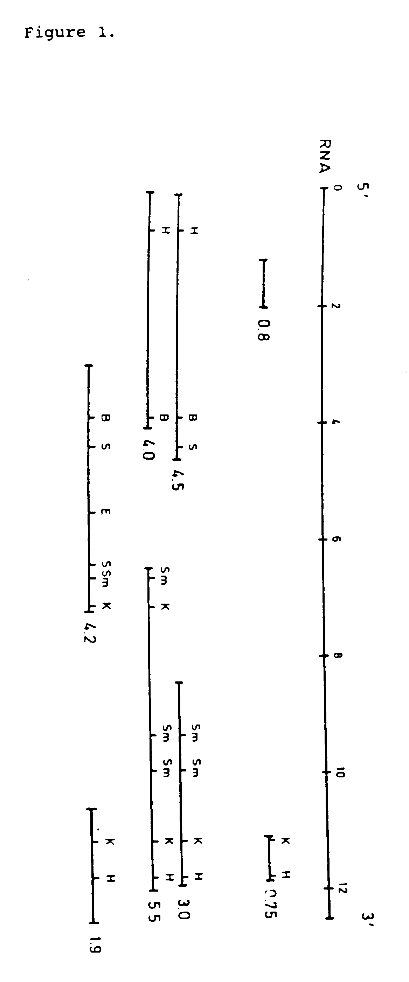

A ³²P 5'-end labeled oligonucleotide of 17 bases complementary to the RNA sequence encoding the amino acid sequence Cys Gly Asp Asp Gly Phe was used for screening a λgt10 cDNA library. This oligonucleotide which hybridized to the about 12 kb genomic RNA of HCV, identified inter alia a clone with an insert of 0.75 kb, which hybridized also to HCV RNA. This 0.75 kb nucleic acid sequence which represents a fragment of the HCV genome together with the 0.8 kb λgt11 nucleic acid sequence insert were used for further library screening resulting in a set of overlapping HCV nucleic acid sequences of which the relative positions and restriction site maps are shown in figure 1. These nucleic acid sequence fragments of the HCV genome are located between the following nucleic acid positions
4.0 kb fragment: 27-4027
4.5 kb fragment: 54-4494
0.8 kb fragment: 1140-2002
4.2 kb fragment: 3246-7252
5.5 kb fragment: 6656-11819
and within about the following nucleic acid positions
3.0 kb fragment: 8920-11920
1.9 kb fragment: 10384-12284
0.75 kb fragment: 10913-11663

Nucleotide sequencing. For complete nucleotide sequence determination exonuclease III and nuclease S1 (enzymes from Boehringer Mannheim, FRG) were used to establish deletion libraries of HCV derived cDNA inserts subcloned into pEMBL 18+ or 19+ plasmids (Hennikoff, 1987). Dideoxy sequencing (Sanger et al. 1977) of single stranded (Dente et al., 1985) or double stranded DNA templates was carried out using the T7 polymerase sequencing kit (Pharmacia, Sweden).

From the cDNA fragments a continuous sequence of 12284 nucleotides in length could be determined as shown in figure 2 (SEQ ID NO: 1). This sequence contains one long open reading frame (ORF), starting with the ATG codon at position 364 to 366 and ending with TGA as a translational stop codon at 12058 to 12060. This ORF consists of 3898 codons capable of encoding a 435 kDa protein with an amino acid sequence shown in figure 2 (SEQ ID NO: 1-2). Three nucleotide exchanges were detected as a result of differences in nucleotide sequence caused by possible heterogenicity of the virus population, two of which resulted in changes in the deduced amino acid sequence (figure 2, SEQ ID NO: 1-2).

It is concluded that almost the complete HCV genome has been cloned and sequenced by the procedures described above.

The 0.8 kb λgt11 nucleic acid sequence encoding an immunogenic HCV polypeptide identified with anti HCV serum was partially sequenced (see figure 3, SEQ ID NO: 8-9) which revealed that this sequence is located within 1.2 and 2.0 kb on the HCV RNA.

### Example 3

### Molecular cloning and expression of fusion proteins of HCV

cDNA fragments derived from two regions of the HCV genome, i.e. the 0,8 kb λgt11 insert of example 1 encoding amino acids 262-546 (see figure 2, SEQ ID NO: 1-2) and the nucleic acid sequence encoding amino acids 747-1071 (figure 2, SEQ ID NO: 1-2), are expressed as fusion proteins in the pEx system (Strebel, K. et al., 1986).

Bacterial extracts were separated by SDS-PAGE and stained according to standard procedures, and then tested for reactivity with the goat anti-HCV serum of example 1 in a Western blot.

The HCV specific fusion proteins were partially purified by SDS-PAGE and transfered to nitrocellulose and incubated with the goat anti-HCV serum. Specific antibodies against said fusion proteins were obtained after elution.

Antibodies specific for the above-mentioned fusion proteins were employed in a radio-immuno precipitation assay.

### Results

Both fusion proteins expressed in the pEx system were clearly identified as HCV specific after reaction with the goat anti-HCV serum.

Monospecific antiserum prepared against both fusions proteins precipitated HCV glycoproteins.

Antibodies specific for the 262-546-fusion protein precipitated the 44/48 kD and 33 kD protein, antibodies specific for the 747-1071-fusion protein precipitated the 55 kD protein from virus infected cells.

### Example 4

### Molecular cloning and expression of structural proteins via vaccinia virus

A fragment of the 4,0 kb clone shown in figure 1 (pHCK11) is prepared starting at the HinfI restriction site (nucleotide 372) and ending at an artificial EcoRI site (nucleotide 4000) (Maniatis et al. 1982). For the 5' end an oligonucleotide adaptor was synthesized which contained an overhang compatible to BamHI, the original ATG(364-366) as translational start codon and a protruding end compatible to HinfI at the 3' end (SEQ ID NO: 5 and 6). At the 3' end of the construct a translational stop codon was introduced by deletion of the EcoRI protruding end with Mung bean nuclease and ligation into a blunt-end StuI/EcoRI adaptor residue (SEQ ID NO: 7): (Maniatis et al. 1982).

Prior to inserting above-mentioned HCV sequences into vaccinia virus the heterologous gene is cloned into a recombination vector. For this purpose a pGS62 plasmid (Cranage, M.P. et al. 1986) was used which contains a cloning site downstream the P7.5K promotor within the 4.9kb thymidine kinase sequence. The cloning site comprises three unique restriction sites, BamHI, SmaI and EcoRI. The recombination vector pGS62-3.8 was established by ligation of the described HCV sequence (372-4000) together with the adaptors into the BamHI/EcoRI digested pGS62.

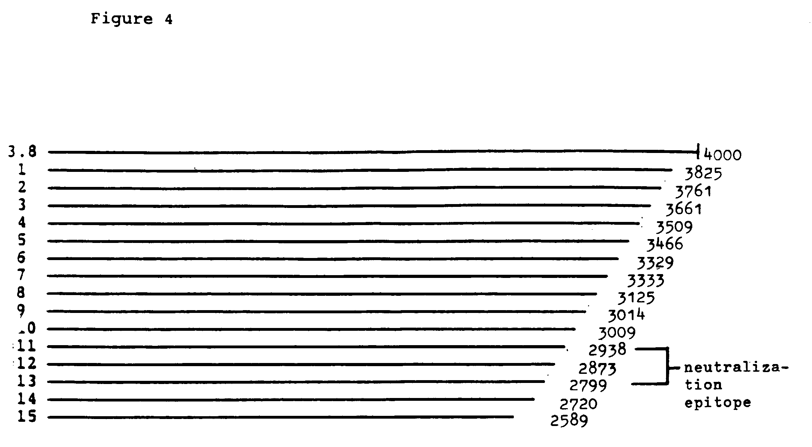

Based on the plasmid a set of 15 deletion mutants was established. By treatment with ExonucleaseIII (Hennikof et al., 1987) subsequent shortening of the HCV cDNA from the 3' end was performed. All deletions are located within the region coding for the HCV 55 kD protein by removal of about 100bp; most of the 55 kD protein is lost in mutant 15 ending at nucleotide 2589. ExoIII shortened cDNA clones were ligated into the pGS62 giving rise to pGS62-3.8Exo 1-15 (figure 4).

CVI cells were infected with vaccinia (strain Copenhagen, mutant TS7) at a MOI of 0.1. Three hours after infection pGS62-3.8 DNA as well as vaccinia WR DNA were transfected by the Ca₃(PO₄)₂ precipitation method and incubated for two days. Virus progeny was harvested and selected for tk-phenotype on 143 tk-cells in the presence of brom-deoxy-Uridine (100 µg/ml). This selection was performed at least twice followed by two further cycles of plaque purification.

### Characterization of vaccinia-HCV recombinants

CVI cells were infected at an MOI between 2 and 10 with vaccinia-HCV recombinants and incubated for 8-16 hours. After fixation of the cells indirect immunofluorescence was performed using either monoclonal antibodies specific for HCV 55 kD protein or polyvalent anti-HCV sera. In all cases a cytoplasmatic fluorescence could be demonstrated.

After radioimmunoprecipitation and western blot analysis of cells infected with vaccinia recombinants four HCV-specific proteins were detected. By labeling with [³H] glucosamine it was shown that three of these proteins are glycosylated. The apparent molecular weights of these proteins were identical to those found in HCV infected cells with HCV specific sera, namely 20 kD(core), 44/48 kD, 33 kD and 55 kD.

Proteolytic processing and modifications appear to be authentic since HCV proteins produced by expression via vaccinia virus have the same apparent molecular weights as in HCV infected cells.

### Induction of neutralizing antibodies against HCV in mice.

Four groups of mice (3 mice/group) were infected once with

| | | | |
|---|---|---|---|
| a. | Vaccinia WR wildtype | (5x10⁶pfu/individual) | **WR** |
| b. | Vaccinia 3.8 recombinant | (5x10⁷pfu/individual) | **VAC3.8** |
| c. | Vaccinia 3.8Exo 4 (55 kD deleted) | (5x10⁷pfu/individual) | **VAC3.8Exo 4** |
| d. | Vaccinia 3.8Exo 5 | (5x10⁷pfu/individual) | **VAC3.8Exo 5** |
| e. | Vaccinia 3.8Exo 15 (55 kD deleted) | (5x10⁷pfu/individual) | **VAC3.8Exo 15** |

by injection of purified virus intraperitoneally.
Mice were bled three weeks later. The reactivity of the sera was checked in a virus neutralization assay with HCV (Alfort) on PK[15] cells after serial dilution.
(Rümenapf, T. et al. 1989).

### Neutralization titers

a. WR <1:2
b. VAC3.8 1:96
c. VAC3.8Exo 4 1:96
d. VAC3.8Exo 5 <1:2
e. VAC3.8Exo 15 <1.2

From the above it can be concluded that vaccinia virus containing a nucleic acid sequence comprising the genetic information for all structural proteins (VAC3.8) is able to induce virus neutralizing antibodies in mice, while incomplete constructs VAC3.8Exo 5-15 and WR are not.

As all deletions are located within the region coding for HCV 55 kD protein (most of the 55 kD protein is lost in mutant 15 ending at nucleotide 2589) and the other structural proteins are still being expressed by the recombinant vaccinia virus, it is clear that the 55 kD protein is responsible for the induction of HCV neutralizing antibodies.

### Example 5

### Immunization of pigs with VAC3.8

Out of three piglets (about 20 kg in weight) one animal (no. 28) was infected with wild type vaccinia virus (WR strain) and the other two (no. 26, 27) with recombinant VAC3.8 (i.p., i.v. and i.d., respectively). For infection 1x10⁸ pfu of vaccinia virus is applied to each animal.

Clinical signs in the course of vaccinia infection were apparent as erythema at the side of scarification and fever (41 °C) at day six after infection.

### Titers against vaccinia and hog cholera virus:

Three weeks after infection the reactivity of the respective sera against vaccinia (WR on CVI cells) and HCV (Alfort on PK15 cells) was checked.

Neutralization was assayed after serial dilution of the sera by checking for complete absence of cpe (vaccinia) or specific signals in immunofluorescence (HCV). (Rümenapf, T. et al. 1989).

### Neutralization titers against vaccinia:

- pig 28 (WR): 1:8
- pig 26 (VAC3.8): 1:16
- pig 27 (VAC3.8): 1:16

### Neutralization titers against HCV:

- pig 28 (WR): <1:2
- pig 26 (VAC3.8): 1:32
- pig 27 (VAC3.8): 1:16

### Challenge with HCV:

Four weeks after immunization with vaccinia each of the pigs was challenged by infection with 5x10⁷ TCID₅₀ HCV Alfort. Virus was applicated oronasal according to the natural route of infection. This amount of virus has been experimentally determined to be compulsory lethal for pigs.

On day five after the challenge infection pig 28 revealed fever of 41.5 °C and kept this temperature until day 12. The moribund animal was killed that day expressing typical clinical signs of acute hog cholera.

Both pigs (26, 27) immunized with VAC3.8 did not show any sign of illness after the challenge with HCV for more than 14 days.

### Example 6

### Construction of a 55 kD protein expression vector

Clone pHCK11 is digested with restriction enzymes SacI and HpaI according to standard techniques.

The resulting 1.3 kb fragment, located between nucleotides 2672 (AGCTC) and 3971 (GTT) comprising most of HCV 55 kD protein, is isolated and cloned into the pseudorabies virus (PRV) gX gene (Maniatis et al. 1982).

Briefly, the cloned gX sequence was digested with SacI and ApaI. The ApaI 5' protruding ends were made blunt by filling up with Klenow fragment. After ligation the putative gX leader peptide coding sequence was located just upstream of the inserted HCV 55 kD sequence.

A translational stop codon downstream the HCV sequence was introduced by digestion with Bgl II (Bgl II site: 3936-3941) and religation after filling up the overhangs with Klenow fragment. This construct was placed downstream of the PRV gX promotor (clone 16/4-1.3). Clone 16/4-1.3 was transfected into MDBK cells by the DEAE dextran method (Maniatis et al. 1989). 16 h. later cells were infected with PRV (m.o.i.=1). 4 h. post infection cells were fixed with a mixture of cold (-20 ^{o}C) methanol/acetone. Indirect immunofluorescence with monoclonal antibodies (MABs) anti-HCV 55 kD protein revealed a specific signal in 5-10% of the cells. PRV infected cells without transfection and cells only transfected with clone 16/4-1.3 did not show any signal in this assay.

### Brief description of the drawings

Fig. 1 displays physical maps of different HCV derived cDNA clones and their position relative to the RNA genome (upper line). Two HCV derived cDNA clones isolated after screening with either the antibody probe (0.8 kb clone) or the degenerated oligonucleotide probe (0.75 kb clone) are shown in the second line. The cDNA fragments chosen for nucleotide sequencing are indicated below. All numbers represent sizes of DNA fragments in kb. Restriction sites: B = Bgl II; E = EcoRI; H = Hind III; K = Kpn I; S = Sal I; Sm = Sma I.

Fig. 2 depicts a nucleic acid sequence of HCV and deduced amino acid sequence of the long open reading frame. Nucleotide exchanges between different cDNA clones and resulting changes in amino acid sequence are indicated. The part of the sequence corresponding to the oligonucleotide used for screening is underlined.

Fig. 3 shows the cDNA sequence from part of the 0.8 kb HCV insert of a λgt11 clone and the deduced amino acid sequence in one-letter code.

Fig. 4 shows the length of the HCV DNA cloned in the pGS62 vector. A set of 15 deletion mutants derived from cDNA clone pHCK11 was established by treatment with Exonuclease III and cloned in the pGS62 vector giving rise to pGS62-3.8Exo 1-15. 3' end nucleotides are indicated.

### References

BENTON, W., and DAVIS, R. (1977). Screening λgt recombinant clones by hybridization to single plaques in sity. Science 196, 180-182.

CHIRGWIN, J.M., PRZYBYLA, A.E., MAcDONALD, R.J., and RUTTER, W.J. (1979). Isolation of biologically active ribonucleic acid from sources enriched in ribonuclease, Biochemistry 18, 5294-5299.

CRANAGE, M.P. et al. (1986). EMBO, J. 5, 3057-3063.

DAVIS, L.G., DIBNER, M.D., and BATTEY, J.F. (1986). Basic Methods in Molecular Biology, 190-191. Elsevier, New York, Amsterdam, London.

DENTE, L., SOLLAZZO, M., BALDARI, C., CESARENI, G., and CORTESE, R. (1985). The pEMBL family of single-stranded vectors. In: DNA Cloning, Vol. 1, (Glover, D.M., ed.), IRL Press Oxford/Washington DC, pp. 101-107.

HENNIKOFF, S. (1987). Unidirectional digestion with exonuclease III in DNA sequence analysis. In: Meth. Enzymol. (Wu, R., ed.) 155, 156-165.

HUYNH, T.V., YOUNG, R.A., and DAVIS, R.W. (1985). Constructing and screening cDNA libraries in λgt10 and λgt11. In: DNA Cloning: A Practical Approach, Vol. 2, (Glover, D.M., ed.), IRL Press Oxford, pp. 49-78.

KEIL, G.M., EBELING-KEIL, A., and KOSZINOWSKI, U.H. (1984). Temporal regulation of murine cytomegalovirus transcription and mapping of viral RNA synthesized at immediate early times after infection, J. Virol. 50, 784-795.

MANIATIS, T., FRITSCH, E.F., and SAMBROOKS, S. (1982). Molecular Cloning, a Laboratory Manual. Cold Spring Harbor Laboratory Press, Cold Spring Harbor, NY.

SANGER, F., NICKLEN, S., and COULSON, A.R. (1977). DNA sequencing with chain-terminating inhibitors. Proc. Natl. Acad. Sci. U.S.A. 74, 5363-5467.

MANIATIS, T. et al. (1989). Molecular Cloning, a Laboratory Manual, second edition, Cold Spring Harbor Laboratory Press, Cold Spring Harbor, NY.

STREBEL, K. et al. (1986). J. Virology 57, 983-991.

RüMENOPF, T. et al. (1989). Virology 171, 18-27.

YOUNG, R.A., and DAVIS, R.W. (1983). Efficient isolation of genes by using antibody probes. Proc. Natl. Acad. Sci. U.S.A. 80, 1194-1198.

## Claims (Claims for the following Contracting State(s): BE, CH, DE, DK, FR, GB, IT, LI, NL, SE)

1. A nucleic acid sequence corresponding to a fragment of the hog cholera virus genome, and encoding a hog cholera virus polypeptide or an antigenic fragment thereof, capable of inducing virus neutralizing antibodies, which polypeptide comprises the amino acid sequence located within the amino acid position 263-487 shown in SEQ ID NO: 2 or is a functional equivalent thereof.

2. A nucleic acid sequence according to claim 1, characterized in that it comprises either the nucleotide sequence 1150-1824 shown in SEQ ID NO: 1 or a fragment thereof, or a nucleotide sequence which hybridizes under stringent conditions to said nucleotide sequence shown in SEQ ID NO: 1.

3. A recombinant nucleic acid molecule comprising a vector nucleic acid molecule and a nucleic acid sequence according to claim 1 or 2.

4. A host cell capable of producing a polypeptide of hog cholera virus comprising a recombinant nucleic acid molecule according to claim 3.

5. A host cell according to claim 4, characterized in that the host cell is a bacterium.

6. A recombinant virus containing a recombinant nucleic acid molecule according to claim 3.

7. A hog cholera virus polypeptide which polypeptide corresponds with a portion of the hog cholera virus precursor polyprotein, capable of inducing virus neutralizing antibodies, which polypeptide comprises the amino acid sequence located within the amino acid position 263-487 shown in SEQ ID NO: 2 or is an antigenic fragment or a functional equivalent thereof.

8. A hog cholera virus polypeptide encoded by a nucleic acid sequence according to claim 1 or 2.

9. A polypeptide according to claim 7 or 8,
characterized in that it contains one or more post-translational modifications.

10. A vaccine for the protection of animals against hog cholera virus infection, characterized in that it comprises a polypeptide according to claims 7-9.

11. A vaccine for the protection of animals against hog cholera virus infection, characterized in that it comprises a host cell according to claims 4-5 or a recombinant virus according to claim 6.

12. A method for the preparation of a hog cholera virus vaccine, characterized in that a polypeptide according to claims 7-9 is formed to a pharmaceutical preparation with immunizing activity.

13. A method for the preparation of a hog cholera virus vaccine, characterized in that a host cell according to claims 4-5 or a recombinant virus according to claim 6 is propagated in a culture, whereafter the host cell or the recombinant virus is harvested and is formed to a pharmaceutical preparation with immunizing activity.

14. Use of a polypeptide expressed by a host cell according to claims 4 or 5, or a recombinant virus according to claim 6 in a diagnostic method to detect the presence of HCV antigen or antibody.

## Claims (Claims for the following Contracting State(s): GR, ES)

1. A process for the preparation of a recombinant nucleic acid molecule which comprises incorporating a nucleic acid sequence corresponding to a fragment of the hog cholera virus genome, and encoding a hog cholera virus polypeptide or an antigenic fragment thereof, capable of inducing virus neutralizing antibodies, which polypeptide comprises the amino acid sequence located within the amino acid position 263-487 shown in SEQ ID No. 2 or is a functional equivalent thereof, in a vector nucleic acid molecule.

2. A process according to claim 1, characterized in that said nucleic acid sequence comprises, either the nucleotide sequence 1150-1824 shown in SEQ ID No. 1 or a fragment thereof, or a nucleotide sequence which hybridizes under stringent conditions to said nucleotide sequence shown in SEQ ID No. 1.

3. A process which comprises expressing in a host cell the nucleic acid sequence defined in claim 1 or 2.

4. A process according to claims 3, characterized in that the host cell is a bacterium.

5. A process for the preparation of a recombinant virus, characterized in that the nucleic acid sequence defined in claim 1 or 2 is incorporated into a virus.

6. A process according to claims 3 or 4 which comprises allowing expression of the nucleic acid sequence in a culture, and recovering the polypeptide from the culture.

7. A process for the preparation of a vaccine for the protection of animals against hog cholera virus infection comprising, following the process of claim 6, the forming of the polypeptide expression product to a pharmaceutical preparation with immunizing activity.

8. A process for the preparation of a vaccine for the protection of animals against hog cholera virus infection comprising forming a host cell capable of expressing the nucleic acid sequence defined in claims 1 or 2, or a recombinant virus comprising the nucleic acid sequence defined in claims 1 or 2, to a pharmaceutical preparation with immunizing activity.

## Patentansprüche (Patentansprüche für folgende(n) Vertragsstaat(en): BE, CH, DE, DK, FR, GB, IT, LI, NL, SE)

1. Nukleinsäuresequenz, die einem Fragment des Schweinepestvirusgenoms entspricht und die für ein Polypeptid eines Schweinepestvirus oder ein antigenes Fragment davon codiert, welches fähig ist virusneutralisierende Antikörper zu induzieren, wobei das Polypeptid die in SEQ ID Nr.: 2 dargestellte Aminosäuresequenz zwischen Aminosäureposition 263-487 umfasst oder ein funktionelles Fragment davon ist.

2. Nukleinsäuresequenz gemäss Anspruch 1, dadurch gekennzeichnet, dass sie entweder die in SEQ ID Nr.: 1 dargestellte Aminosäuresequenz 1150-1824 oder ein Fragment davon umfasst oder eine Nukleotidsequenz, die unter stringenten Bedingungen mit genannter in SEQ ID Nr. 1 dargestellter Nukleotidsequenz hybridisiert.

3. Rekombinantes Nukleinsäuremolekül, welches ein Vektornukleinsäuremolekül und eine Nukleinsäuresequenz gemäss Anspruch 1 oder 2 umfasst.

4. Wirtszelle, die fähig ist ein Polypeptid des Schweinepestvirus zu produzieren, welche ein Nukleinsäuremolekül gemäss Anspruch 3 umfasst.

5. Wirtszelle gemäss Anspruch 4, dadurch gekennzeichnet, dass die Wirtszelle ein Bakterium ist.

6. Rekombinantes Virus, das ein rekombinantes Nukleinsäuremolekül gemäss Anspruch 3 enthält.

7. Polypeptid eines Schweinepestvirus, welches einem Teil des Vorläuferpolyproteins des Schweinepestvirus entspricht, das fähig ist virusneutralisierende Antikörper zu induzieren, wobei das Polypeptid die in SEQ ID Nr.:2 dargestellte Aminosäuresequenz zwischen Aminosäureposition 263-487 umfasst oder ein antigenes Fragment oder ein funktionales Fragment davon ist.

8. Polypeptid eines Schweinepestvirus , welches von der Nukleinsäuresequenz gemäss Anspruch 1 oder 2 codiert wird.

9. Polypeptid gemäss Anspruch 7 oder 8, dadurch gekennzeichnet, dass es eine oder mehrere posttranlationale Modifikationen enthält.

10. Impfstoff zum Schutz von Tieren gegen Schweinepestvirusinfektion, dadurch gekennzeichnet, dass er ein Polypeptid gemäss gemäss Ansprüchen 7-9 enthält.

11. Impfstoff zum Schutz von Tieren gegen Schweinepestvirusinfektion, dadurch gekennzeichnet, dass er eine Wirtszelle gemäss Ansprüchen 4-5 oder ein rekombinantes Virus gemäss Anspruch 6 umfasst.

12. Verfahren zur Herstellung eines Impfstoffes gegen das Schweinepestvirus, dadurch gekennzeichnet, dass aus einem Polypeptid gemäss Ansprüchen 7-9 ein pharmazeutisches Präparat mit immunisierender Aktivität hergestellt wird.

13. Verfahren zur Herstellung eines Impfstoffes gegen das Schweinepestvirus, dadurch gekennzeichnet, dass eine Wirtszelle gemäss Ansprüchen 4-5 oder ein rekombinantes Virus gemäss Anspruch 6 in einer Kultur vermehrt wird, wonach die Wirtszelle oder das rekombinante Virus geerntet und daraus ein pharmazeutisches Präparat mit immunisierender Wirkung hergestellt wird.

14. Verwendung des von einer Wirtszelle gemäss Ansprüchen 4 oder 5 exprimierten Polypeptids oder eines rekombinanten Virus gemäss Anspruch 6 in einem diagnostischen Verfahren zum Nachweis der Anwesenheit von HCV-Antigen oder Antikörper.

## Patentansprüche (Patentansprüche für folgende(n) Vertragsstaat(en): GR, ES)

1. Verfahren zur Herstellung eines rekombinanten Nukleinsäuremoleküls, das den Einbau einer einem Fragment des Schweinepestvirusgemoms entsprechenden Nukleinsäuresequenz umfasst und für ein Polypeptid eines Schweinepestvirus oder ein antigenes Fragment davon codiert, welches fähig ist virusneutralisierende Antikörper zu induzieren, wobei das Polypeptid die in SEQ ID. Nr. 2 dargestellte Aminosäuresequenz innerhalb der Aminosäureposition 263-487 umfasst oder ein funktionales Äquivalent davon in einem Vektornukleinsäuremolekül ist.

2. Verfahren gemäss Anspruch 1, dadurch gekennzeichnet, dass genannte Nukleinsäuresequenz entweder die in SEQ ID Nr. 1 dargestellte Nukleotidsequenz 1150-1824 oder ein Fragment davon umfasst oder eine Nukleotidsequenz, die unter stringenten Bedingungen mit genannter in SEQ ID Nr.1 dargestellter Nukleotidsequenz hybridisiert.

3. Verfahren, welches das Exprimieren der in Anspruch 1 oder 2 definierten Nukleinsäuresequenz in einer Wirtszelle umfasst.

4. Verfahren gemäss Anspruch 3, dadurch gekennzeichnet, dass die Wirtszelle ein Bakterium ist.

5. Verfahren zur Herstellung eines rekombinanten Virus, dadurch gekennzeichnet, dass die in Anspruch 1 oder 2 definierte Nukleinsäuresequenz in ein Virus inkorporiert wird.

6. Verfahren gemäss Anspruch 3 oder 4, welches die Expression der Nukleinsäuresequenz in einer Kultur und die Gewinnung des Polypeptids aus der Kultur umfasst.

7. Verfahren zur Herstellung eines Impfstoffes zum Schutz von Tieren gegen Schweinepestvirusinfektion, welches, dem Verfahren von Anspruch 6 folgend, das Herstellen eines pharmazeutischen Präparats mit immunisierender Aktivität aus dem Polypeptidexpressionsprodukt umfasst.

8. Verfahren zur Herstellung eines Impfstoffes zum Schutz von Tieren gegen Schweinepestvirusinfektion, welches das Herstellen eines pharmazeutischen Präparats mit immunisierender Aktivität. aus einer Wirtszelle umfasst, die fähig ist, die in den Ansprüchen 1 oder 2 definierte Nukleinsäuresequenz zu exprimieren oder aus einem rekombinanten Virus, das die in Ansprüchen 1 oder 2 definierte Nukleinsäuresequenz umfasst.

## Revendications (Revendications pour l'(les) Etat(s) contractant(s) suivant(s): BE, CH, DE, DK, FR, GB, IT, LI, NL, SE)

1. Une séquence d'acide nucléique correspondant à un fragment du génome du virus de la peste porcine, et codant pour un polypeptide du virus de la peste porcine ou un fragment antigénique de ce dernier, capable d'induire la synthèse anticorps neutralisant le virus, lequel polypeptide comprend la séquence d'acides aminés localisée entre les positions d'acide aminé 263-487, décrite DANS SEQ ID NO:2 ou correspond à un équivalent fonctionnel de ce dernier.

2. Une séquence d'acide nucléique selon la revendication 1, caractérisée en ce qu'elle comprend soit la séquence nucléotidique 1150-1824 décrite dans SEQ ID NO:1 ou un fragment de cette dernière, soit une séquence nucléotidique qui s'hybride dans des conditions drastiques à ladite séquence nucléotidique décrite dans SEQ ID NO:1.

3. Une molécule d'acide nucléique recombinante comprenant une molécule d'acide nucléique vecteur et une séquence nucléotidique selon la revendication 1 ou 2.

4. Une cellule hôte capable de produire un polypeptide du virus de la peste porcine comprenant une molécule d'acide nucléique recombinante selon la revendication 3.

5. Une cellule hôte selon la revendication 4, caractérisée en ce que la cellule hôte est une bactérie.

6. Un virus recombinant contenant une molécule d'acide nucléique recombinant selon la revendication 3.

7. Un polypeptide du virus de la peste porcine lequel polypeptide correspond à une partie de la polyprotéine précurseur du virus de la peste porcine, capable d'induire la synthèse d'anticorps neutralisant le virus, lequel polypeptide comprend la séquence d'acides aminés localisée entre les positions d'acide aminé 263-487 décrite dans SEQ ID NO:2 ou correspond à un fragment antigénique ou à un équivalent antigénique de ce dernier.

8. Un polypeptide du virus de la peste porcine codé par une séquence d'acide nucléique selon la revendication 1 ou 2.

9. Un polypeptide selon la revendication 7 ou 8 caractérisé en ce qu'il contient une ou plusieurs modifications posttraductionnelles.

10. Un vaccin pour la protection des animaux contre une infection par le virus de la peste porcine caractérisé en ce qu'il comprend un polypeptide selon les revendications 7 à 9.

11. Un vaccin pour la protection des animaux contre une infection par le virus de la peste porcine caractérisé en ce qu'il comprend une cellule hôte selon les revendications 4 à 5 ou un virus recombinant selon la revendication 6.

12. Un procédé de préparation d'un vaccin contre le virus de la peste porcine caractérisé en ce qu'un polypeptide selon les revendications 7-9 est transformé en une préparation pharmaceutique présentant une activité immunisante.

13. Un procédé de préparation d'un vaccin contre le virus de la peste porcine caractérisé en ce que une cellule hôte selon les revendications 4 à 5 ou un virus recombinant selon la revendication 6 est propagé dans une culture, puis la cellule hôte ou le virus recombinant est récolté et transformé en une préparation pharmaceutique présentant une activité immunisante.

14. Utilisation d'un polypeptide exprimé par une cellule hôte selon l'une des revendications 4 ou 5, ou d'un virus recombinant selon la revendication 6, dans une méthode de diagnostic pour détecter la présence d'antigène ou d'anticorps du HCV.

## Revendications (Revendications pour l'(les) Etat(s) contractant(s) suivant(s): GR, ES)

1. Un procédé de préparation d'une molécule d'acide nucléique recombinante qui comprend l'incorporation d'une séquence nucléotidique correspondant à un fragment du génome du virus de la peste porcine, et codant pour un polypeptide du virus de la peste porcine ou un fragment antigénique de ce dernier, capable d'induire des anticorps neutralisant le virus, lequel polypeptide comprend la séquence d'acides aminés localisée entre les positions d'acide aminé 263-487 décrite dans SEQ ID NO:2 ou correspond à un équivalent fonctionnel de ce dernier, dans une molécule d'acide nucléique vecteur.

2. Un procédé selon la revendication 1 caractérisé en ce que ladite séquence nucléotidique comprend soit la séquence nucléotidique 1150-1824 décrite dans SEQ ID NO:1 ou un fragment de cette dernière, soit une séquence nucléotidique qui s'hybride dans des conditions drastiques à ladite séquence nucléotidique décrite dans SEQ ID NO:1.

3. Un procédé qui comprend l'expression dans une cellule hôte de la séquence d'acide nucléique définie dans la revendication 1 ou 2.

4. Un procédé selon la revendication 3, caractérisé en ce que la cellule hôte est une bactérie.

5. Un procédé de préparation d'un virus recombinant caractérisé en ce que la séquence d'acide nucléique définie dans la revendication 1 ou 2 est incorporée dans un virus.

6. Un procédé selon la revendication 3 ou 4 qui comprend l'expression de la séquence d'acide nucléique dans une culture et la récupération du polypeptide à partir de la culture.

7. Un procédé de préparation d'un vaccin pour la protection des animaux contre une infection par le virus de la peste porcine comprenant, en suivant le procédé de la revendication 6, la transformation du produit d'expression du polypeptide en une préparation pharmaceutique présentant une activité immunisante.

8. Un procédé de préparation d'un vaccin pour la protection des animaux contre une infection par le virus de la peste porcine comprenant la transformation d'une cellule hôte capable d'exprimer la séquence d'acide nucléique définie dans la revendication 1 ou 2, ou d'un virus recombinant comprenant la séquence d'acide nucléique définie dans la revendication 1 ou 2, en une composition pharmaceutique présentant une activité immunisante.
